# EUROPÄISCHE PATENTSCHRIFT

(11) **EP 3 137 492 B1**
(45) Veröffentlichungstag und Bekanntmachung des Hinweises auf die Patenterteilung: **17.10.2018**
(21) Anmeldenummer: 15717860.9
(22) Anmeldetag: 16.04.2015
(51) Int. Cl.: C07K 14/435

(54) **VERFAHREN ZUR PRODUKTION VON L-AMINOSÄUREN IN CORYNEBAKTERIEN UNTER VERWENDUNG EINES GLYCIN SPALTENDEN SYSTEMS**
METHOD FOR PRODUCING L-AMINO ACIDS IN CORYNEBACTERIA USING A GLYCINE CLEAVAGE SYSTEM
PROCÉDÉ DE PRODUCTION D'ACIDES AMINÉS L DANS DES BACTÉRIES CORYNE AU MOYEN D'UN SYSTÈME DE CLIVAGE DE LA GLYCINE

(30) Priorität: 30.04.2014 EP 14166649
(43) Veröffentlichungstag der Anmeldung: 08.03.2017
(73) Patentinhaber: Evonik Degussa GmbH, 45128 Essen (DE)
(72) Erfinder: OCHROMBEL, Ines, 33729 Bielefeld (DE); BATHE, Brigitte, 33154 Salzkotten (DE); HASSELMEYER, Marleen, 33098 Paderborn (DE)
(86) Internationale Anmeldenummer: PCT/EP2015/058280
(87) Internationale Veröffentlichungsnummer: WO 2015/165740

(56) Entgegenhaltungen:
- WO-A2-2008/101857
- CHUN-YUAN WU ET AL: "Corynebacterium humireducens sp. nov., an alkaliphilic, humic acid-reducing bacterium isolated from a microbial fuel cell", INTERNATIONAL JOURNAL OF SYSTEMATIC AND EVOLUTIONARY MICROBIOLOGY, SOCIETY FOR GENERAL MICROBIOLOGY, READING, GB, Bd. 61, Nr. 4, 1. April 2011 (2011-04-01), Seiten 882-887, XP002692942, ISSN: 1466-5026, DOI: 10.1099/IJS.0.020909-0 [gefunden am 2010-05-21]

## Beschreibung

Die vorliegende Erfindung betrifft ein Verfahren zur Produktion von L-Aminosäuren in Corynebakterien, in welchem ein Glycin spaltendes System eingesetzt wird.

Verfahren zur Produktion von L-Aminosäuren, in welchen Bakterien der Gattung Corynebacterium eingesetzt werden, sind dem Fachmann bekannt.

Obwohl zahlreiche Corynebacterium-Arten bekannt sind, werden in diesen Verfahren üblicherweise Bakterien der Art Corynebacterium glutamicum eingesetzt, da sich diese Art als besonders vorteilhaft für die Produktion von L-Aminosäuren herausgestellt hat.

Ferner wurde herausgefunden, dass durch die Verwendung eines Glycin spaltenden Systems (GCV) die Ausbeute an L-Aminosäure weiter gesteigert werden kann, da durch das Glycin spaltende System die Bildung des unerwünschten Nebenprodukts L-Glycin weitgehend unterbunden werden kann. Die Unterdrückung der Bildung von L-Glycin bzw. der Abbau überschüssigen L-Glycins ist insbesondere bei der Produktion von L-Methionin von besonderer Bedeutung, da hier L-Glycin als äquimolares Nebenprodukt anfällt.

Ein Glycin spaltendes System besteht aus mehreren Untereinheiten, nämlich den Untereinheiten GcvP, GcvT und GcvH. Es handelt sich um einen Multi-Enzym-Komplex, der die oxidative Decarboxylierung und Deaminierung von Glycin zu Kohlendioxid, Ammoniumionen und N⁵⁻¹⁰-Methylen-tetrahydrofolat katalysiert.

Bei der Glycin-Dehydrogenase GcvP handelt es sich um eine Pyridoxalphosphat enthaltende Decarboxylase, die Kohlendioxid freisetzt und die Aminomethyl-Verbindung gebunden an Pyridoxalphosphat zurücklässt.

Bei dem H-Protein GcvH handelt es sich um eine Lipoamid enthaltende Aminomethyltransferase.

Das Enzym GcvT katalysiert den nukleophilen Angriff des Aminomethyllipoamids durch Tetrahydrofolat unter Ausbildung von N⁵⁻¹⁰-methylen-tetrahydrofolat und Freisetzung von Ammoniumionen, wobei vollständig reduziertes Lipoamid gebunden an GcvH zurückbleibt.

Glycin spaltende Systeme kommen nicht in allen Corynebakterien vor, sondern sind bisher nur für sehr wenige Corynebakterien beschrieben worden. So besitzt beispielsweise insbesondere der für die Aminosäureproduktion bevorzugte Produktionsstamm C. glutamicum kein eigenes Glycin spaltendes System. Um die Vorteile eines Glycin spaltenden Systems für C. glutamicum nutzbar zu machen, musste ein solches daher aus einem anderen Corynebakterium in C. glutamicum eingebaut und heterolog exprimiert werden. Verwendet hierfür wurde bislang das Glycin spaltende System aus C. jeikeium (WO 2008/101857). Ein gravierender Nachteil ist hierbei, dass es sich bei C. jeikeium um einen pathogenen Organismus handelt. Weiterer Nachteil ist, dass zwecks Abbau des unerwünschten Nebenprodukts Glycin ein heterologes Konstrukt hergestellt werden musste.

Primäre Aufgabe der vorliegenden Erfindung war es daher, ein neues Glycin spaltendes System zur Verfügung zu stellen, vorzugsweise ein Glycin spaltendes System, das nicht aus einem pathogenen Organismus stammt, wobei das Glycin spaltende System vorzugsweise zum Einbau in andere Corynebakterien, insbesondere C. glutamicum, geeignet ist.

Weitere Aufgabe der vorliegenden Erfindung war es, ein Corynebakterium zur Verfügung zu stellen, das von Hause aus dazu in der Lage ist, L-Aminosäuren zu produzieren und darüber hinaus über ein eigenes Glycin spaltendes System verfügt, so dass die Herstellung eines heterologen Konstrukts - wie im Falle von C. glutamicum - nicht erforderlich ist.

Erfindungsgemäß wurde nun überraschenderweise gefunden, dass die Art Corynebacterium humireducens, die nicht pathogen ist, über ein eigenes Glycin spaltendes System verfügt, das sich strukturell wesentlich von den bisher beschriebenen Glycin spaltenden Systemen unterscheidet.

Überraschenderweise wurde ferner gefunden, dass das Glycin spaltende System aus Corynebacterium humireducens sehr effektiv ist, so dass im Wildtypstamm Glycin, das ein unerwünschtes Nebenprodukt bei der Aminosäuresynthese darstellt, in der Zelle nicht oder nur in geringer Menge akkumuliert. Durch Verstärkung eines erfindungsgemäßen Glycin spaltenden Systems, insbesondere durch Überexpression, kann die Unterdrückung der Glycin-Nebenproduktbildung entsprechend noch weiter verbessert werden. Insbesondere kann durch Überexpression eines erfindungsgemäßen Glycin spaltenden Systems in anderen Mikroorganismen, insbesondere in C. glutamicum, die Glycin-Nebenproduktbildung ebenfalls wirksam unterdrückt werden.

Weiterhin wurde überraschenderweise gefunden, dass C. humireducens bereits von Hause aus dazu in der Lage ist, L-Aminosäuren, insbesondere L-Alanin, L-Valin und L-Glutaminsäure, über den eigenen Bedarf hinaus zu produzieren, so dass C. humireducens einen L-Aminosäure-Produktionsstamm mit einem homologen Glycin spaltenden System darstellt, der insbesondere als Ausgangspunkt für die Entwicklung weiterer Produktionsstämme, die über ein wirtseigenes Glycin spaltenden System verfügen, herangezogen werden kann.

Der Stamm C. humireducens wird zum ersten Mal beschrieben durch Wu et al. (International Journal of Systematic and Evolutionary Microbiology (2011), 61, 882-887). Er wurde bei der DSMZ hinterlegt unter der Hinterlegungsnummer DSM 45392, seine 16S rRNA wurde bei EMBL hinterlegt und hat die Zugangsnummer GQ421281. Bei dem Ausgangsstamm handelt es sich um ein halotolerantes, alkaliphiles, Huminsäure reduzierendes Bakterium.

Weitere Informationen über C. humireducens sind folgenden Publikationen zu entnehmen: Wu et al. (Microb. Biotechnol. (2013), 6(2), 141-149), Lin et al. (Bioresour. Technol. (2013), 136, 302-308).

Ein erster Gegenstand der vorliegenden Erfindung sind daher die Enzyme eines Glycin spaltenden Systems ausgewählt aus der Gruppe bestehend aus:
a) ein Enzym GcvP mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 40,
b) ein Enzym GcvT mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 42,
c) ein Enzym GcvH mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 38.

Ein weiterer Gegenstand der vorliegenden Erfindung ist daher ein Glycin spaltendes System umfassend die Enzyme GcvP, GcvT und GcvH, dadurch gekennzeichnet, dass es mindestens eines der folgenden Polypeptide umfasst:
a) ein Enzym GcvP mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 40,
b) ein Enzym GcvT mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 42,
c) ein Enzym GcvH mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 38.

Vorzugsweise umfasst ein erfindungsgemäßes Glycin spaltendes System hierbei mindestens zwei, vorzugsweise alle drei, der zuvor genannten Polypeptide (a) bis (c).

Besonders bevorzugt ist hierbei ein Glycin spaltendes System, das folgende drei Polypeptide umfasst:
a) ein Enzym GcvP mit einer Sequenzidentität von mindestens 95 oder 98 % zu der Sequenz gemäß SEQ ID NO: 40,
b) ein Enzym GcvT mit einer Sequenzidentität von mindestens 95 oder 98 % zu der Sequenz gemäß SEQ ID NO: 42,
c) ein Enzym GcvH mit einer Sequenzidentität von mindestens 95 oder 98 % zu der Sequenz gemäß SEQ ID NO: 38.

Zu dem Glycin spaltenden System gehören im weiteren Sinne auch die Enzyme LpdA, LplA, LipA, LipB und GcvH.

Die Dihydrolipoamid-Dehydrogenase(LpdA) reoxidiert das an GcvH gebundene Lipoamid.

Die Lipoat-Protein-Ligase A (LplA) katalysiert die Lipoylierung des GcvH.

Die Liponsäure-Synthase (LipA) katalysiert die Synthese von Liponsäure.

Die Lipoyl-[Acyl-Carrier-Protein]-Protein-N-Lipoyltransferase (LipB) katalysiert den Transfer der Liponsäure auf das GcvH.

Bei GcvL handelt es sich um eine Dihydrolipoyl-Dehydrogenase.

In einer bevorzugten Ausführungsform umfasst ein erfindungsgemäßes Glycin spaltendes System daher weiterhin mindestens ein weiteres Enzym ausgewählt aus der Gruppe bestehend aus:
a) ein Enzym LipA, vorzugsweise ein Enzym LipA mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 48,
b) ein Enzym LipB, vorzugsweise ein Enzym LipB mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 50,
c) ein Enzym Lpd, vorzugsweise ein Enzym Lpd mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 52,
d) ein Enzym LplA, vorzugsweise ein Enzym LplA mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 94,
e) ein Enzym GcvL, vorzugsweise ein Enzym GcvL mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 96.

In einer erfindungsgemäß besonders bevorzugten Ausführungsform umfasst ein erfindungsgemäßes Glycin spaltendes System hierbei mindestens zwei, drei oder vier, vorzugsweise alle fünf, der zuvor genannten Polypeptide.

Alternativ oder zusätzlich zum Einsatz entsprechender Enzyme, die für eine ausreichende Synthese von Liponsäure und/oder Liponamid sorgen, können diese Verbindungen auch dem Reaktionsmedium zugegeben werden, beispielsweise in Mengen von bis zu 15 mM.

Weiterer Gegenstand der vorliegenden Erfindung sind ebenso Polynukleotide, die für erfindungsgemäße Enzyme und/oder ein erfindungsgemäßes Glycin spaltendes System kodieren. Diese Polynukleotide sind ausgewählt aus der Gruppe bestehend aus:
a) ein Polynukleotid (gcvP), das für das Enzym GcvP kodiert und eine Sequenzidentität von mindestens 70 oder 75 %, vorzugsweise mindestens 80 oder 85 %, insbesondere mindestens 90 oder 95 %, vor allem mindestens 98 oder 100 %, zu der Sequenz von Position 301 bis 3162 gemäß SEQ ID NO: 39 aufweist und/oder unter stringenten Bedingungen mit einem Polynukleotid hybridisiert, dessen Sequenz komplementär zu der Sequenz von Position 301 bis 3162 gemäß SEQ ID NO: 39 ist;
b) ein Polynukleotid (gcvT), das für das Enzym GcvT kodiert und eine Sequenzidentität von mindestens 70 oder 75 %, vorzugsweise mindestens 80 oder 85 %, insbesondere mindestens 90 oder 95 %, vor allem mindestens 98 oder 100 %, zu der Sequenz von Position 301 bis 1404 gemäß SEQ ID NO: 41 aufweist und/oder unter stringenten Bedingungen mit einem Polynukleotid hybridisiert, dessen Sequenz komplementär zu der Sequenz von Position 301 bis 1404 gemäß SEQ ID NO: 41 ist;
c) ein Polynukleotid (gcvH), das für das Enzym GcvH kodiert und eine Sequenzidentität von mindestens 70 oder 75 %, vorzugsweise mindestens 80 oder 85 %, insbesondere mindestens 90 oder 95 %, vor allem mindestens 98 oder 100 %, zu der Sequenz von Position 301 bis 699 gemäß SEQ ID NO: 37 aufweist und/oder unter stringenten Bedingungen mit einem Polynukleotid hybridisiert, dessen Sequenz komplementär zu der Sequenz von Position 301 bis 699 gemäß SEQ ID NO: 37 ist.

Unter "stringenten Bedingungen" ist erfindungsgemäß zu verstehen Waschen bei einer Salzkonzentration von 1 x SSC und 0,1 Gew.-% SDS bei einer Temperatur von 80°C.

Weiterer Gegenstand der vorliegenden Erfindung sind entsprechend auch Vektoren, insbesondere Klonierungs- und Expressionsvektoren, die mindestens ein erfindungsgemäßes Polynukleotid enthalten. Diese Vektoren können entsprechend in Mikroorganismen, insbesondere in coryneforme Bakterien, vor allem der Gattung Corynbebacterium, oder Enterobacteriaceae, vor allem der Gattung Escherichia, eingebaut werden.

Erfindungsgemäße Vektoren können ein oder mehrere erfindungsgemäße Polynukleotide enthalten. Ein erfindungsgemäß bevorzugter Vektor enthält mindestens ein Polynukleotid, das für ein erfindungsgemäßes Enzym ausgewählt aus GcvP, GcvT und GcvH kodiert. Ein besonders bevorzugter Vektor enthält Polynukleotide, die für alle drei erfindungsgemäßen Enzyme GcvP, GcvT und GcvH kodieren.

Erfindungsgemäße Vektoren verfügen vorzugsweise über einen geeigneten Promotor und/oder geeignete Promotoren sowie gegebenenfalls weitere regulatorische Elemente, die die Expression erfindungsgemäßer Polynukleotide in dem rekombinanten Bakterium, vorzugsweise dem rekombinanten Corynebakterium, ermöglichen.

Erfindungsgemäße Polynukleotide können weiterhin zwecks Expression der kodierten Gene auch in das Genom von Mikroorganismen, insbesondere in das Genom von coryneformen Bakterien, insbesondere solchen der Gattung Corynebacterium, oder in das Genom von Enterobacteriaceae, vor allem der Gattung Escherichia, eingebaut werden.

Weiterer Gegenstand der vorliegenden Erfindung sind entsprechend auch rekombinante Mikroorganismen, vorzugsweise Bakterien, insbesondere coryneforme Bakterien, vor allem solche der Gattung Corynebacterium, besonders bevorzugt der Art C. humireducens oder C. glutamicum, sowie Enterobacteriaceae, vor allem solche der Gattung Escherichia, die mindestens ein erfindungsgemäßes Enzym und/oder mindestens ein erfindungsgemäßes Polynukleotid und/oder mindestens einen erfindungsgemäßen Vektor und/oder ein erfindungsgemäßes Glycin spaltendes System und/oder für ein erfindungsgemäßes Glycin spaltendes System kodierende Polynukleotide enthalten.

Ein bevorzugter Gegenstand sind hierbei rekombinante Corynebakterien, insbesondere der Art C. humireducens und der Art C. glutamicum, die mindestens ein erfindungsgemäßes Enzym, vorzugsweise alle erfindungsgemäßen Enzyme, ausgewählt aus GcvP, GcvT und GcvH, und/oder für diese kodierende Polynukleotide und/oder mindestens einen diese Polynukleotide umfassenden Vektor enthalten.

Ein besonderer Gegenstand der vorliegenden Erfindung sind insbesondere auch rekombinante Mikroorganismen, vorzugsweise Bakterien, insbesondere coryneforme Bakterien, vor allem solche der Gattung Corynebacterium mit Ausnahme der Art C. humireducens, insbesondere solche der Art C. glutamicum, die mindestens ein erfindungsgemäßes Enzym und/oder mindestens ein erfindungsgemäßes Polynukleotid und/oder mindestens einen erfindungsgemäßen Vektor und/oder ein erfindungsgemäßes Glycin spaltendes System und/oder für ein erfindungsgemäßes Glycin spaltendes System kodierende Polynukleotide enthalten.

Ein bevorzugter Gegenstand sind hierbei rekombinante Corynebakterien mit Ausnahme der Art C. humireducens, insbesondere der Art C. glutamicum, die mindestens ein erfindungsgemäßes Enzym, vorzugsweise alle erfindungsgemäßen Enzyme, ausgewählt aus GcvP, GcvT und GcvH, und/oder für diese kodierende Polynukleotide und/oder mindestens einen diese Polynukleotide umfassenden Vektor enthalten.

Unter einem "rekombinanten Mikroorganismus" bzw. "rekombinanten Bakterium" ist erfindungsgemäß ein Mikroorganismus bzw. Bakterium zu verstehen, das mindestens einer gentechnischen Maßnahme unterzogen wurde. Bei der gentechnischen Maßnahme kann es sich hierbei insbesondere um eine zielgerichtete oder ungerichtete Mutation, den Einbau eines wirtsfremden Gens und/oder die Überexpression oder Abschwächung eines wirtseigenen oder wirtsfremden Gens handeln. Vorzugsweise zeichnet sich ein erfindungsgemäßer rekombinanter Mikroorganismus bzw. ein erfindungsgemäßes rekombinantes Bakterium durch die Überexpression oder Abschwächung mindestens eines Gens aus. In einer besonders bevorzugten Ausführungsform zeichnet sich ein erfindungsgemäßer rekombinanter Mikroorganismus bzw. ein erfindungsgemäßes rekombinantes Bakterium durch die Überexpression mindestens eines erfindungsgemäßen Enzyms und/oder des für dieses kodierenden Polynukleotids und/oder die Überexpression eines erfindungsgemäßen Glycin spaltenden Systems bzw. der für dieses kodierenden Polynukleotide aus.

Unter "geringer Menge" ist in Bezug auf die Glycin-Bildung vorzugsweise eine Menge von höchstens 0,3 g/l, vorzugsweise von höchstens 0,2 oder 0,1 g/l, besonders bevorzugt von höchstens 0,05 oder höchstens 0,03 g/l, jeweils bezogen auf den Gehalt an akkumuliertem Glycin in der Zelle und/oder im Fermentationsmedium nach Beendigung der Fermentation zu verstehen.

Innerhalb der Gattung Corynebacterium werden erfindungsgemäß Stämme bevorzugt, die auf folgenden Arten beruhen: Corynebacterium efficiens, wie zum Beispiel der Typstamm DSM44549, Corynebacterium glutamicum, wie zum Beispiel der Typstamm ATCC13032 oder der Stamm R, Corynebacterium ammoniagenes, wie zum Beispiel der Stamm ATCC6871, Corynebacterium humireducens, wie zum Beispiel der Stamm DSM 45392, sowie Corynebacterium pekinese, wie zum Beispiel der Stamm CGMCC No. 5361.

Besonders bevorzugt sind die Arten Corynebacterium glutamicum und Corynebacterium humireducens. Sofern im Rahmen dieser Anmeldung vom Stamm Corynebacterium humireducens die Rede ist, handelt es sich vorzugsweise um den Stamm DSM 45392 oder um einen davon abgeleiteten Stamm.

Einige Vertreter der Art Corynebacterium glutamicum sind im Stand der Technik auch unter anderen Bezeichnungen bekannt. Hierzu gehören beispielsweise: Corynebacterium acetoacidophilum ATCC13870, Corynebacterium lilium DSM20137, Corynebacterium melassecola ATCC17965, Brevibacterium flavum ATCC14067, Brevibacterium lactofermentum ATCC13869 und Brevibacterium divaricatum ATCC14020. Der Begriff "Micrococcus glutamicus" für Corynebacterium glutamicum war ebenfalls gebräuchlich. Einige Vertreter der Art Corynebacterium efficiens wurden im Stand der Technik auch als Corynebacterium thermoaminogenes bezeichnet, wie zum Beispiel der Stamm FERM BP-1539.

Angaben zur taxonomischen Einordnung von Stämmen der Gruppe der coryneformen Bakterien findet man unter anderem bei Seiler (Journal of General Microbiology 129, 1433-1477 (1983)), Kinoshita (1985, Glutamic Acid Bacteria, p 115-142. In: Demain and Solomon (ed), Biology of Industrial Microorganisms. The Benjamin/Cummins Publishing Co., London, UK), Kämpfer und Kroppenstedt (Canadian Journal of Microbiology 42, 989-1005 (1996)), Liebl et al (International Journal of Systematic Bacteriology 41, 255-260 (1991)), Fudou et al (International Journal of Systematic and Evolutionary Microbiology 52, 1127-1131 (2002)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "NRRL" können von der Agricultural Research Service Patent Culture Collection (ARS, Peoria, Illinois, US) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Stämme mit der Bezeichnung "CGMCC" können vom China General Microbiological Culture Collection Center (CGMCC, Beijing, China) bezogen werden.

Weiterer Gegenstand der vorliegenden Erfindung ist ebenso ein Verfahren zur Überproduktion einer L-Aminosäure, dadurch gekennzeichnet, dass in diesem Verfahren mindestens ein erfindungsgemäßes Enzym und/oder ein für dieses kodierendes Polynukleotid und/oder ein erfindungsgemäßes Glycin spaltendes System und/oder für dieses kodierende Polynukleotide und/oder ein erfindungsgemäßer rekombinanter Mikroorganismus, vorzugsweise ein erfindungsgemäßes rekombinantes Bakterium, insbesondere ein erfindungsgemäßes rekombinantes coryneformes Bakterium, besonders bevorzugt ein erfindungsgemäßes rekombinantes Corynebakterium, vor allem ein Corynebacterium der Art. C. humireducens oder C. glutamicum, eingesetzt wird. In einer erfindungsgemäß bevorzugten Ausführungsform wird hierbei das mindestens eine erfindungsgemäße Polynukleotid in überexprimierter Form eingesetzt.

Ein bevorzugter Gegenstand der vorliegenden Erfindung ist hierbei ein Verfahren zur Überproduktion einer L-Aminosäure, dadurch gekennzeichnet, dass in diesem Verfahren ein Glycin spaltendes System umfassend die Enzyme GcvP, GcvT und GcvH eingesetzt wird, wobei das Glycin spaltende System mindestens eines der erfindungsgemäßen Enzyme GcvP, GcvT und GcvH umfasst, und/oder in diesem Verfahren für die Enzyme eines Glycin spaltenden Systems GcvP, GcvT und GcvH kodierende Polynukleotide eingesetzt werden, wobei diese Polynukleotide mindestens eines der erfindungsgemäßen Polynukleotide gcvP, gcvT und gcvH umfassen, und/oder ein rekombinantes Corynebakterium, vorzugsweise der Art C. humireducens oder C. glutamicum, eingesetzt wird, das mindestens ein erfindungsgemäßes Enzym, vorzugsweise alle drei erfindungsgemäßen Enzyme, ausgewählt aus GcvP, GcvT und GcvH und/oder mindestens ein erfindungsgemäßes Polynukleotid, vorzugsweise alle drei erfindungsgemäßen Polynukleotide, ausgewählt aus gcvP, gcvT und gcvH enthält.

Ein besonders bevorzugter Gegenstand der vorliegenden Erfindung ist hierbei ein Verfahren zur Überproduktion einer L-Aminosäure, dadurch gekennzeichnet, dass in diesem Verfahren ein Glycin spaltendes System eingesetzt wird, das die erfindungsgemäßen Enzyme GcvP, GcvT und GcvH und/oder für diese Enzyme kodierende Polynukleotide enthält, und/oder in diesem Verfahren ein rekombinantes Corynebacterium, vorzugsweise der Art. C. humireducens oder C. glutamicum, eingesetzt wird, das ein solches Glycin spaltendes System und/oder für dieses kodierende Polynukleotide enthält.

Unter "L-Aminosäure" sind erfindungsgemäß insbesondere die proteinogenen L-Aminosäuren zu verstehen.

Die L-Aminosäure ist hierbei vorzugsweise ausgewählt aus L-Alanin, L-Valin, L-Aminosäuren der Glutamat-Familie, insbesondere L-Glutamat, L-Glutamin, L-Prolin und L-Arginin, sowie L-Aminosäuren der Aspartat-Familie, insbesondere L-Aspartat, L-Asparagin, L-Methionin, L-Lysin, L-Isoleucin und L-Threonin, besonders bevorzugt ausgewählt aus L-Alanin, L-Valin, L-Glutamat, L-Methionin, L-Lysin und L-Threonin. Besonders bevorzugt handelt es sich um L-Methionin.

Die Überproduktion der L-Aminosäure erfolgt erfindungsgemäß vorzugsweise in C. humireducens oder C. glutamicum.

Erfindungsgemäße Verfahren zeichnen sich vorzugsweise dadurch aus, dass nur geringe Mengen an Glycin-Nebenprodukt anfallen. Glycin fällt in erfindungsgemäßen Verfahren vorzugsweise in einer Menge von weniger als 0,2 g/l, insbesondere in einer Menge von weniger als 0,1 g/l, besonders bevorzugt in einer Menge von weniger als 0,05 g/l an.

Unter "Überproduzieren" bzw. "Überproduktion" ist erfindungsgemäß in Bezug auf die L-Aminosäure zu verstehen, dass die Mikroorganismen die L-Aminosäure über ihren eigenen Bedarf hinaus produzieren und entweder in der Zelle anreichern oder in das sie umgebende Nährmedium ausscheiden und dort akkumulieren. Vorzugsweise sind die Mikroorganismen hierbei dazu in der Lage ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l der betreffenden L-Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren.

Erfindungsgemäße rekombinante Mikroorganismen, in die erfindungsgemäße Polynukleotide und/oder erfindungsgemäße Vektoren eingebracht wurden, besitzen in einer bevorzugten Ausführungsform bereits vor dem Einbau der erfindungsgemäßen Polynukleotide und/oder Vektoren die Fähigkeit eine L-Aminosäure überzuproduzieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist offensichtlich und bedarf keiner weiteren Erklärungen, dass man zu einem erfindungsgemäßen rekombinanten Mikroorganismus auch dadurch gelangen kann, indem man einen Wildstamm, in welchem ein erfindungsgemäßes Polynukleotid und/oder ein erfindungsgemäßer Vektor enthalten ist oder eingebaut wurde, anschließend durch geeignete weitere genetische Maßnahmen dazu veranlasst, die L-Aminosäure zu produzieren bzw. die L-Aminosäure-Produktion zu erhöhen.

Überexpression der entsprechenden Polynukleotide bzw. Polypeptide kann die Aminosäureproduktion bestimmter L-Aminosäuren positiv beeinflusst werden.

Gegenstand der vorliegenden Erfindung sind daher ebenso:
a) eine Threonindehydratase (IlvA, EC 4.3.1.19) mit einer Sequenzidentität von mindestens 90, 95 oder 98 %, vorzugsweise 100 %, zu der Sequenz gemäß SEQ ID NO: 106 sowie für diese kodierende Polynukleotide,
b) die Untereinheit einer Acetolactat-Synthase (IlvB), die eine Sequenzidentität von mindestens 90, 95 oder 98 %, vorzugsweise 100 %, zu der Sequenz gemäß SEQ ID NO: 98, aufweist, sowie für diese kodierende Polynukleotide,
c) eine Isomeroreduktase (IlvC, EC 1.1.1.86) mit einer Sequenzidentität von mindestens 90, 95 oder 98 %, vorzugsweise 100 %, zu der Sequenz gemäß SEQ ID NO: 100 sowie für diese kodierende Polynukleotide,
d) eine Dihydroxy-acid Dehydratase (IlvD, EC 4.2.1.9) mit einer Sequenzidentität von mindestens 90, 95 oder 98 %, vorzugsweise 100 %, zu der Sequenz gemäß SEQ ID NO: 102 sowie für diese kodierende Polynukleotide,
e) eine Transaminase (IlvE, EC 2.6.1.42) mit einer Sequenzidentität von mindestens 90, 95 oder 98 %, vorzugsweise 100 %, zu der Sequenz gemäß SEQ ID NO: 104 sowie für diese kodierende Polynukleotide,
f) eine Acetolactat-Synthase (IlvH, EC 2.2.1.6) mit einer Sequenzidentität von mindestens 90, 95 oder 98 %, vorzugsweise 100 %, zu der Sequenz gemäß SEQ ID NO: 122 sowie für diese kodierende Polynukleotide,
g) eine 3-Methyl-2-Oxobutanoat-Hydroxymethyltransferase (PanB, EC 2.1.2.11) mit einer Sequenzidentität von mindestens 90, 95 oder 98 %, vorzugsweise 100 %, zu der Sequenz gemäß SEQ ID NO: 118 sowie für diese kodierende Polynukleotide,
h) eine Pantothenat-Synthase (PanC, EC 6.3.2.1) mit einer Sequenzidentität von mindestens 90, 95 oder 98 %, vorzugsweise 100 %, zu der Sequenz gemäß SEQ ID NO: 120 sowie für diese kodierende Polynukleotide,
i) eine Glutamat-Dehydrogenase (Gdh) mit einer Sequenzidentität von mindestens 90, 95 oder 98 %, vorzugsweise 100 %, zu der Sequenz gemäß SEQ ID NO: 124 sowie für diese kodierende Polynukleotide,
j) eine Glutamin-Synthetase (Glutamin-Synthetase 1) mit einer Sequenzidentität von mindestens 90, 95 oder 98 %, vorzugsweise 100 %, zu der Sequenz gemäß SEQ ID NO: 126 sowie für diese kodierende Polynukleotide,
k) eine Glutamin-Synthetase (Glutamin-Synthetase 2) mit einer Sequenzidentität von mindestens 90, 95 oder 98 %, vorzugsweise 100 %, zu der Sequenz gemäß SEQ ID NO: 128 sowie für diese kodierende Polynukleotide,
l) eine Glutamat-Synthase mit einer Sequenzidentität von mindestens 90, 95 oder 98 %, vorzugsweise 100 %, zu der Sequenz gemäß SEQ ID NO: 130 sowie für diese kodierende Polynukleotide,
m) eine Isocitrat-Dehydrogenase mit einer Sequenzidentität von mindestens 90, 95 oder 98 %, vorzugsweise 100 %, zu der Sequenz gemäß SEQ ID NO: 132 sowie für diese kodierende Polynukleotide,
n) eine Aconitat-Hydrase mit einer Sequenzidentität von mindestens 90, 95 oder 98 %, vorzugsweise 100 %, zu der Sequenz gemäß SEQ ID NO: 134 sowie für diese kodierende Polynukleotide,
o) eine Citrat-Synthase mit einer Sequenzidentität von mindestens 90, 95 oder 98 %, vorzugsweise 100 %, zu der Sequenz gemäß SEQ ID NO: 136 sowie für diese kodierende Polynukleotide,
p) eine Aminopeptidase C (PepC) mit einer Sequenzidentität von mindestens 90, 95 oder 98 %, vorzugsweise 100 %, zu der Sequenz gemäß SEQ ID NO: 138 sowie für diese kodierende Polynukleotide,
q) eine Pyruvat-Dehydrogenase mit einer Sequenzidentität von mindestens 90, 95 oder 98 %, vorzugsweise 100 %, zu der Sequenz gemäß SEQ ID NO: 140 sowie für diese kodierende Polynukleotide,
r) eine Pyruvat-Kinase (Pyruvat-Kinase 1) mit einer Sequenzidentität von mindestens 90, 95 oder 98 %, vorzugsweise 100 %, zu der Sequenz gemäß SEQ ID NO: 142 sowie für diese kodierende Polynukleotide,
s) eine Pyruvat-Kinase (Pyruvat-Kinase 2) mit einer Sequenzidentität von mindestens 90, 95 oder 98 %, vorzugsweise 100 %, zu der Sequenz gemäß SEQ ID NO: 144 sowie für diese kodierende Polynukleotide,
t) eine Enolase mit einer Sequenzidentität von mindestens 90, 95 oder 98 %, vorzugsweise 100 %, zu der Sequenz gemäß SEQ ID NO: 146 sowie für diese kodierende Polynukleotide,
u) eine 2,3-Bisphosphoglycerat-abhängige Phosphoglycerat-Mutase (GpmA) mit einer Sequenzidentität von mindestens 90, 95 oder 98 %, vorzugsweise 100 %, zu der Sequenz gemäß SEQ ID NO: 148 sowie für diese kodierende Polynukleotide,
v) eine Phosphoglycerat-Kinase (Pgk) mit einer Sequenzidentität von mindestens 90, 95 oder 98 %, vorzugsweise 100 %, zu der Sequenz gemäß SEQ ID NO: 150 sowie für diese kodierende Polynukleotide,
w) eine Glycerinaldehyd-3-phosphat-Dehydrogenase (Glycerin-3-phosphat-Dehydrogenase 1) mit einer Sequenzidentität von mindestens 90, 95 oder 98 %, vorzugsweise 100 %, zu der Sequenz gemäß SEQ ID NO: 152 sowie für diese kodierende Polynukleotide,
x) eine Glycerinaldehyd-3-phosphat-Dehydrogenase (Glycerin-3-phosphat-Dehydrogenase 2) mit einer Sequenzidentität von mindestens 90, 95 oder 98 %, vorzugsweise 100 %, zu der Sequenz gemäß SEQ ID NO: 154 sowie für diese kodierende Polynukleotide,
y) eine Triosephosphat-Isomerase (TpiA) mit einer Sequenzidentität von mindestens 90, 95 oder 98 %, vorzugsweise 100 %, zu der Sequenz gemäß SEQ ID NO: 156 sowie für diese kodierende Polynukleotide,
z) eine Fructosebisphosphataldolase mit einer Sequenzidentität von mindestens 90, 95 oder 98 %, vorzugsweise 100 %, zu der Sequenz gemäß SEQ ID NO: 158 sowie für diese kodierende Polynukleotide,
aa) eine 1-Phosphofructokinase mit einer Sequenzidentität von mindestens 90, 95 oder 98 %, vorzugsweise 100 %, zu der Sequenz gemäß SEQ ID NO: 160 sowie für diese kodierende Polynukleotide,
bb) eine 6-Phosphofructokinase mit einer Sequenzidentität von mindestens 90, 95 oder 98 %, vorzugsweise 100 %, zu der Sequenz gemäß SEQ ID NO: 162 sowie für diese kodierende Polynukleotide,
cc) eine Homoserinkinase (ThrB, EC 2.7.1.39) mit einer Sequenzidentität von mindestens 90, 95 oder 98 %, vorzugsweise 100 %, zu der Sequenz gemäß SEQ ID NO: 4 sowie für diese kodierende Polynukleotide,
dd) eine Cystein-Synthase (CBS, CysK) mit einer Sequenzidentität von mindestens 90, 95 oder 98 %, vorzugsweise 100 %, zu der Sequenz gemäß SEQ ID NO: 22 sowie für diese kodierende Polynukleotide,
ee) eine Cystathionin-Betalyase (AecD) mit einer Sequenzidentität von mindestens 90, 95 oder 98 %, vorzugsweise 100 %, zu der Sequenz gemäß SEQ ID NO: 26 sowie für diese kodierende Polynukleotide,
ff) eine Aspartat-Semialdehyd-Dehydrogenase (Asd, EC 1.2.1.11) mit einer Sequenzidentität von mindestens 90, 95 oder 98 %, vorzugsweise 100 %, zu der Sequenz gemäß SEQ ID NO: 28 sowie für diese kodierende Polynukleotide,
gg) die kleinere Untereinheit eines Transporters für verzweigt-kettige Aminosäuren (BrnE) mit einer Sequenzidentität von mindestens 90, 95 oder 98 %, vorzugsweise 100 %, zu der Sequenz gemäß SEQ ID NO: 30 sowie für diese kodierende Polynukleotide,
hh) die größere Untereinheit eines Transporters für verzweigt-kettige Aminosäuren (BrnF) mit einer Sequenzidentität von mindestens 90, 95 oder 98 %, vorzugsweise 100 %, zu der Sequenz gemäß SEQ ID NO: 32 sowie für diese kodierende Polynukleotide,
ii) eine Serin-Acetyltransferase (CysE) mit einer Sequenzidentität von mindestens 90, 95 oder 98 %, vorzugsweise 100 %, zu der Sequenz gemäß SEQ ID NO: 34 sowie für diese kodierende Polynukleotide,
jj) eine Cystein-Synthase (CysK) mit einer Sequenzidentität von mindestens 90, 95 oder 98 %, vorzugsweise 100 %, zu der Sequenz gemäß SEQ ID NO: 36 sowie für diese kodierende Polynukleotide,
kk) eine Serin-Hydroxymethyltransferase (GlyA) mit einer Sequenzidentität von mindestens 90, 95 oder 98 %, vorzugsweise 100 %, zu der Sequenz gemäß SEQ ID NO: 44 sowie für diese kodierende Polynukleotide,
ll) eine gegebenenfalls feedbackresistente Homoserin-Dehydrogenase (Hom, EC 1.2.1.11) mit einer Sequenzidentität von mindestens 90, 95 oder 98 %, vorzugsweise 100 %, zu der Sequenz gemäß SEQ ID NO: 46 sowie für diese kodierende Polynukleotide,
mm) eine gegebenenfalls feedbackresistente Aspartat-Kinase (LysC, EC 2.7.2.4) mit einer Sequenzidentität von mindestens 90, 95 oder 98 %, vorzugsweise 100 %, zu der Sequenz gemäß SEQ ID NO: 54 sowie für diese kodierende Polynukleotide,
nn) eine Cystathionin-gamma-Synthase (MetB) mit einer Sequenzidentität von mindestens 90, 95 oder 98 %, vorzugsweise 100 %, zu der Sequenz gemäß SEQ ID NO: 56 sowie für diese kodierende Polynukleotide,
oo) eine 5,10-Methylentetrahydrofolat-Reduktase (MetF) mit einer Sequenzidentität von mindestens 90, 95 oder 98 %, vorzugsweise 100 %, zu der Sequenz gemäß SEQ ID NO: 58 sowie für diese kodierende Polynukleotide,
pp) eine Homoserin-O-Acetyltransferase (MetX) mit einer Sequenzidentität von mindestens 90, 95 oder 98 %, vorzugsweise 100 %, zu der Sequenz gemäß SEQ ID NO: 60 sowie für diese kodierende Polynukleotide,
qq) eine O-Acetylhomoserin-Lyase (MetY) mit einer Sequenzidentität von mindestens 90, 95 oder 98 %, vorzugsweise 100 %, zu der Sequenz gemäß SEQ ID NO: 62 sowie für diese kodierende Polynukleotide,
rr) eine gegebenenfalls feedbackresistente Pyruvat-Carboxylase (Pyc, EC 6.4.1.1) mit einer Sequenzidentität von mindestens 90, 95 oder 98 %, vorzugsweise 100 %, zu der Sequenz gemäß SEQ ID NO: 64 sowie für diese kodierende Polynukleotide,
ss) eine gegebenenfalls feedback-resistente D-3-Phosphoglycerat-Dehydrogenase (SerA) mit einer Sequenzidentität von mindestens 90, 95 oder 98 %, vorzugsweise 100 %, zu der Sequenz gemäß SEQ ID NO: 66 sowie für diese kodierende Polynukleotide,
tt) eine Phosphoserin-Phosphatase (SerB) mit einer Sequenzidentität von mindestens 90, 95 oder 98 %, vorzugsweise 100 %, zu der Sequenz gemäß SEQ ID NO: 68 sowie für diese kodierende Polynukleotide,
uu) eine Phosphoserin-Aminotransferase (SerC) mit einer Sequenzidentität von mindestens 90, 95 oder 98 %, vorzugsweise 100 %, zu der Sequenz gemäß SEQ ID NO: 70 sowie für diese kodierende Polynukleotide,
vv) die Untereinheit einer Sulfat-Adenylyltransferase (CysD) mit einer Sequenzidentität von mindestens 90, 95 oder 98 %, vorzugsweise 100 %, zu der Sequenz gemäß SEQ ID NO: 74 sowie für diese kodierende Polynukleotide,
ww) eine Adenosin-Phosphosulfat-Reduktase (CysH) mit einer Sequenzidentität von mindestens 90, 95 oder 98 %, vorzugsweise 100 %, zu der Sequenz gemäß SEQ ID NO: 76 sowie für diese kodierende Polynukleotide,
xx) eine Sulfit-Reduktase (Cysl) mit einer Sequenzidentität von mindestens 90, 95 oder 98 %, vorzugsweise 100 %, zu der Sequenz gemäß SEQ ID NO: 78 sowie für diese kodierende Polynukleotide,
yy) eine NADPH-abhängige Glutamatsynthase-beta-Kette (CysJ) mit einer Sequenzidentität von mindestens 90, 95 oder 98 %, vorzugsweise 100 %, zu der Sequenz gemäß SEQ ID NO: 80 sowie für diese kodierende Polynukleotide,
zz) die große Untereinheit einer Sulfat-Adenylyltransferase (CysN) mit einer Sequenzidentität von mindestens 90, 95 oder 98 %, vorzugsweise 100 %, zu der Sequenz gemäß SEQ ID NO: 82 sowie für diese kodierende Polynukleotide,
aaa) eine Cystathionin-beta-Synthase (CysY) mit einer Sequenzidentität von mindestens 90, 95 oder 98 %, vorzugsweise 100 %, zu der Sequenz gemäß SEQ ID NO: 84, sowie für diese kodierende Polynukleotide,
bbb) ein Sulfat-Transporter (CysZ) mit einer Sequenzidentität von mindestens 90, 95 oder 98 %, vorzugsweise 100 %, zu der Sequenz gemäß SEQ ID NO: 86 sowie für diesen kodierende Polynukleotide,
ccc) eine 5-Methyltetrahydropteroyl-triglutamat-homocystein-Methyltransferase (MetE) mit einer Sequenzidentität von mindestens 90, 95 oder 98 %, vorzugsweise 100 %, zu der Sequenz gemäß SEQ ID NO: 88 sowie für diese kodierende Polynukleotide,
ddd) eine Peptidyl-tRNA-Hydrolase 1 (PtH1) mit einer Sequenzidentität von mindestens 90, 95 oder 98 %, vorzugsweise 100 %, zu der Sequenz gemäß SEQ ID NO: 90 sowie für diese kodierende Polynukleotide,
eee) eine Peptidyl-tRNA-Hydrolase 2 (PtH2) mit einer Sequenzidentität von mindestens 90, 95 oder 98 %, vorzugsweise 100 %, zu der Sequenz gemäß SEQ ID NO: 92, sowie für diese kodierende Polynukleotide,
fff) eine Diaminopimelat-Dehydrogenase (Ddh, EC 1.4.1.16) mit einer Sequenzidentität von mindestens 90, 95 oder 98 %, vorzugsweise 100 %, zu der Sequenz gemäß SEQ ID NO: 202 sowie für diese kodierende Polynukleotide,
ggg) eine Diaminopimelat-Decarboxylase (LysA, EC 4.1.1.20) mit einer Sequenzidentität von mindestens 90, 95 oder 98 %, vorzugsweise 100 %, zu der Sequenz gemäß SEQ ID NO: 164 sowie für diese kodierende Polynukleotide,
hhh) eine Aspartat-Aminotransferase (AaT, EC 2.6.1.1) mit einer Sequenzidentität von mindestens 90, 95 oder 98 %, vorzugsweise 100 %, zu der Sequenz gemäß SEQ ID NO: 166, sowie für diese kodierende Polynukleotide,
iii) ein L-Lysin-Exporter (LysE, Lysin-Efflux-Permease) mit einer Sequenzidentität von mindestens 90, 95 oder 98 %, vorzugsweise 100 %, zu der Sequenz gemäß SEQ ID NO: 168, sowie für diesen kodierende Polynukleotide,
jjj) eine Dihydropicolinat-Reduktase (DapB, EC 1.3.1.26) mit einer Sequenzidentität von mindestens 90, 95 oder 98 %, vorzugsweise 100 %, zu der Sequenz gemäß SEQ ID NO: 170 sowie für diese kodierende Polynukleotide,
kkk) eine Glucose-6-phosphat-Dehydrogenase (EC 1.1.1.49) mit einer Sequenzidentität von mindestens 90, 95 oder 98 %, vorzugsweise 100 %, zu der Sequenz gemäß SEQ ID NO: 172 sowie für diese kodierende Polynukleotide,
lll) die Zwf-Untereinheit einer Glucose-6-phosphat-Dehydrogenase (Zwf, EC 1.1.1.49) mit einer Sequenzidentität von mindestens 90, 95 oder 98 %, vorzugsweise 100 %, zu der Sequenz gemäß SEQ ID NO: 186 sowie für diese kodierende Polynukleotide,
mmm) die OpcA-Untereinheit einer Glucose-6-phosphat-Dehydrogenase (OpcA, EC 1.1.1.49) mit einer Sequenzidentität von mindestens 90, 95 oder 98 %, vorzugsweise 100 %, zu der Sequenz gemäß SEQ ID NO: 188 sowie für diese kodierende Polynukleotide,
nnn) eine Phosphogluconsäure-Dehydrogenase (Gnd, EC 1.1.1.44) mit einer Sequenzidentität von mindestens 90, 95 oder 98 %, vorzugsweise 100 %, zu der Sequenz gemäß SEQ ID NO: 174 sowie für diese kodierende Polynukleotide.

Weiterer Gegenstand der vorliegenden Erfindung sind ebenso Vektoren, die die zuvor genannten Polynukleotide enthalten, sowie rekombinanten Mikroorganismen, die die zuvor genannten Enzyme und/oder Polynukleotide und/oder Vektoren enthalten. Das betreffende Polypeptid und/oder Polynukleotid liegt hierbei in einer bevorzugten Ausführungsform in dem Mikroorganismus in überexprimierter Form vor. Bei den rekombinanten Mikroorganismen handelt es sich hierbei vorzugsweise um coryneforme Bakterien, vor allem um Corynebakterien, insbesondere solchen der Art C. humireducens oder C. glutamicum.

Weiterer Gegenstand der vorliegenden Erfindung ist daher auch ein Verfahren zur Überproduktion einer L-Aminosäure, vorzugsweise ausgewählt aus L-Alanin, L-Valin, L-Aminosäuren der Glutamat-Familie, insbesondere L-Glutamat, L-Glutamin, L-Prolin und L-Arginin, sowie L-Aminosäuren der Aspartat-Familie, insbesondere L-Aspartat, L-Asparagin, L-Methionin, L-Lysin, L-Isoleucin und L-Threonin, besonders bevorzugt ausgewählt aus L-Alanin, L-Valin, L-Glutamat, L-Methionin, L-Lysin und L-Threonin, vor allem zur Überproduktion von L-Methionin, in welchem mindestens eines, vorzugsweise mindestens zwei, drei oder vier, der genannten Polynukleotide in überexprimierter Form vorliegen, wobei das Verfahren vorzugsweise in Corynebakterien, insbesondere solche der Art. C. humireducens oder C. glutamicum, durchgeführt wird.

Weiterer Gegenstand der vorliegenden Erfindung sind auch weitere Polynukleotide aus C. humireducens sowie die durch diese Polynukleotide kodierten Polypeptide. Durch Ausschaltung oder Abschwächung der entsprechenden Polynukleotide bzw. Polypeptide kann die Aminosäureproduktion bestimmter L-Aminosäuren positiv beeinflusst werden.

Gegenstand der vorliegenden Erfindung sind daher ebenso Polypeptide ausgewählt aus der folgenden Liste:
a) eine Threonin-Synthase (ThrC, EC 4.2.3.1) mit einer Sequenzidentität von mindestens 90, 95 oder 98 %, vorzugsweise 100 %, zu der Sequenz gemäß SEQ ID NO: 108 sowie für diese kodierende Polynukleotide,
b) eine Isopropylmalat-Synthase (LeuA, EC 2.3.3.13) mit einer Sequenzidentität von mindestens 90, 95 oder 98 %, vorzugsweise 100 %, zu der Sequenz gemäß SEQ ID NO: 110 sowie für diese kodierende Polynukleotide,
c) eine Isopropylmalat-Dehydrogenase (LeuB, EC 1.1.1.85) mit einer Sequenzidentität von mindestens 90, 95 oder 98 %, vorzugsweise 100 %, zu der Sequenz gemäß SEQ ID NO: 112 sowie für diese kodierende Polynukleotide,
d) die Untereinheiten einer Isopropylmalat-Isomerase (LeuCD, EC 4.2.1.33), die Sequenzidentitäten von mindestens 90, 95 oder 98 %, vorzugsweise 100 %, zu den Sequenzen gemäß SEQ ID NO: 114 bzw. SEQ ID NO: 116 aufweisen sowie für diese kodierende Polynukleotide,
e) die Untereinheiten einer Succinyl-CoA-Ligase (SucCD, EC 6.2.1.5) mit Sequenzidentitäten von jeweils mindestens 90, 95 oder 98 %, vorzugsweise 100 %, zu den Sequenzen gemäß SEQ ID NO: 198 bzw. SEQ ID NO: 200 sowie für diese kodierende Polynukleotide,
f) eine DNA-Bindungsdomäne vom Typ HTH tetR (McbR) mit einer Sequenzidentität von mindestens 90, 95 oder 98 %, vorzugsweise 100 %, zu der Sequenz gemäß SEQ ID NO: 2 sowie für diese kodierende Polynukleotide,
g) eine Homoserinkinase (ThrB, EC 2.7.1.39) mit einer Sequenzidentität von mindestens 90, 95 oder 98 %, vorzugsweise 100 %, zu der Sequenz gemäß SEQ ID NO: 4 sowie für diese kodierende Polynukleotide,
h) eine Glucose-6-phosphat-lsomerase (Pgi, EC 5.3.1.9) mit einer Sequenzidentität von mindestens 90, 95 oder 98 %, vorzugsweise 100 %, zu der Sequenz gemäß SEQ ID NO: 6 sowie für diese kodierende Polynukleotide,
i) eine Phosphoenolpyruvat-Carboxykinase (Pck, EC 4.1.1.32) mit einer Sequenzidentität von mindestens 90, 95 oder 98 %, vorzugsweise 100 %, zu der Sequenz gemäß SEQ ID NO: 8 sowie für diese kodierende Polynukleotide,
j) ein D-Methionin bindendes Lipoprotein (MetQ) mit einer Sequenzidentität von mindestens 90, 95 oder 98 %, vorzugsweise 100 %, zu der Sequenz gemäß SEQ ID NO: 10 sowie für dieses kodierende Polynukleotide,
k) ein Methionin-Transporter (MetP) mit einer Sequenzidentität von mindestens 90, 95 oder 98 %, vorzugsweise 100 %, zu der Sequenz gemäß SEQ ID NO: 12 sowie für diesen kodierende Polynukleotide,
l) ein ATP-abhängiger Methionin-Transporter (MetN) mit einer Sequenzidentität von mindestens 90, 95 oder 98 %, vorzugsweise 100 %, zu der Sequenz gemäß SEQ ID NO: 14 sowie für diesen kodierende Polynukleotide,
m) eine S-Adenosylmethionin-Synthase (MetK) mit einer Sequenzidentität von mindestens 90, 95 oder 98 %, vorzugsweise 100 %, zu der Sequenz gemäß SEQ ID NO: 16 sowie für diese kodierende Polynukleotide,
n) eine Methionin-Importsystem-Permease (Metl) mit einer Sequenzidentität von mindestens 90, 95 oder 98 %, vorzugsweise 100 %, zu der Sequenz gemäß SEQ ID NO: 18 sowie für diese kodierende Polynukleotide,
o) eine 4-Hydroxy-Tetrahydrodipicolinat-Synthase (DapA, EC 4.3.3.7) mit einer Sequenzidentität von mindestens 90, 95 oder 98 %, vorzugsweise 100 %, zu der Sequenz gemäß SEQ ID NO: 20 sowie für diese kodierende Polynukleotide,
p) eine Carboxylat-Amin-Ligase mit einer Sequenzidentität von mindestens 90, 95 oder 98 %, vorzugsweise 100 %, zu der Sequenz gemäß SEQ ID NO: 24 sowie für diese kodierende Polynukleotide,
q) eine Malat-Chinon-Oxidoreduktase (Mqo, EC 1.1.99.16) mit einer Sequenzidentität von mindestens 90, 95 oder 98 %, vorzugsweise 100 %, zu der Sequenz gemäß SEQ ID NO: 176 sowie für diese kodierende Polynukleotide,
r) die E1p-Untereinheit eines Pyruvat-Dehydrogenase-Komplexes (AceE, EC 1.2.4.1) mit einer Sequenzidentität von mindestens 90, 95 oder 98 %, vorzugsweise 100 %, zu der Sequenz gemäß SEQ ID NO: 178 sowie für diese kodierende Polynukleotide,
s) eine Citrat-Synthase (GltA, EC 4.1.3.7) mit einer Sequenzidentität von mindestens 90, 95 oder 98 %, vorzugsweise 100 %, zu der Sequenz gemäß SEQ ID NO: 180 sowie für diese kodierende Polynukleotide,
t) eine Malat-Dehydrogenase (Mdh, EC 1.1.1.37) mit einer Sequenzidentität von mindestens 90, 95 oder 98 %, vorzugsweise 100 %, zu der Sequenz gemäß SEQ ID NO: 182 sowie für diese kodierende Polynukleotide,
u) eine UDP-N-acetylmuramoylalanyl-D-glutamat-2,6-diaminopimelat-Ligase (MurE, EC 6.3.2.13) mit einer Sequenzidentität von mindestens 90, 95 oder 98 %, vorzugsweise 100 %, zu der Sequenz gemäß SEQ ID NO: 184, sowie für diese kodierende Polynukleotide.

Weiterer Gegenstand der vorliegenden Erfindung sind ebenso Vektoren, die die zuvor genannten Polynukleotide enthalten, sowie rekombinanten Mikroorganismen, die die zuvor genannten Enzyme und/oder Polynukleotide und/oder Vektoren enthalten. Das betreffende Polypeptid und/oder Polynukleotid liegt hierbei in einer bevorzugten Ausführungsform in dem Mikroorganismus in ausgeschalteter oder abgeschwächter Form vor. Bei den rekombinanten Mikroorganismen handelt es sich hierbei vorzugsweise um coryneforme Bakterien, vor allem um Corynebakterien, insbesondere solche der Art C. humireducens oder C. glutamicum, vor allem der Art C. humireducens.

In einer weiteren erfindungsgemäß bevorzugten Ausführungsform weisen erfindungsgemäße Mikroorganismen oder Bakterien, insbesondere erfindungsgemäße Corynebakterien, vor allem erfindungsgemäße Corynebakterien der Art C. humireducens oder C. glutamicum, insbesondere erfindungsgemäße L-Methionin-Überproduktionsstämme, neben einem erfindungsgemäßen, vorzugsweise überexprimierten, Glycin spaltenden System bzw. der für dieses kodierenden Polynukleotide, mindestens eines, vorzugsweise mindestens zwei oder drei, besonders bevorzugt mindestens vier oder fünf, der folgenden Merkmale auf:
a) ein abgeschwächtes Polynukleotid (mcbR), das für eine DNA-Bindungsdomäne vom Typ HTH tetR (McbR), vorzugsweise für eine DNA-Bindungsdomäne mit einer Sequenzidentität von mindestens 90, 95 oder 98 %, vorzugsweise 100 %, zu der Sequenz gemäß SEQ ID NO: 2, kodiert,
b) ein abgeschwächtes Polynukleotid (thrB-Gen), das für eine Homoserinkinase (ThrB, EC 2.7.1.39), vorzugsweise für eine Homoserinkinase mit einer Sequenzidentität von mindestens 90, 95 oder 98 %, vorzugsweise 100 %, zu der Sequenz gemäß SEQ ID NO: 4, kodiert,
c) ein abgeschwächtes Polynukleotid (pgi), das für eine Glucose-6-phosphat-lsomerase (Pgi, EC 5.3.1.9), vorzugsweise für eine Glucose-6-phosphat-lsomerase mit einer Sequenzidentität von mindestens 90, 95 oder 98 %, vorzugsweise 100 %, zu der Sequenz gemäß SEQ ID NO: 6 kodiert,
d) ein abgeschwächtes Polynukleotid (pck), das für eine Phosphoenolpyruvat-Carboxykinase (Pck, EC 4.1.1.32), vorzugsweise für eine Phosphoenolpyruvat Carboxykinase mit einer Sequenzidentität von mindestens 90, 95 oder 98 %, vorzugsweise 100 %, zu der Sequenz gemäß SEQ ID NO: 8 kodiert,
e) ein abgeschwächtes Polynukleotid (metQ), das für ein D-Methionin bindendes Lipoprotein (MetQ), vorzugsweise für ein D-Methionin bindendes Lipoprotein mit einer Sequenzidentität von mindestens 90, 95 oder 98 %, vorzugsweise 100 %, zu der Sequenz gemäß SEQ ID NO: 10 kodiert,
f) ein abgeschwächtes Polynukleotid (metP), das für einen Methionin-Transporter (MetP), vorzugsweise für einen Methionin-Transporter mit einer Sequenzidentität von mindestens 90, 95 oder 98 %, vorzugsweise 100 %, zu der Sequenz gemäß SEQ ID NO: 12 kodiert,
g) ein abgeschwächtes Polynukleotid (metN), das für einen ATP-abhängigen Methionin-Transporter (MetN), vorzugsweise für einen ATP-abhängigen Methionin-Transporter mit einer Sequenzidentität von mindestens 90, 95 oder 98 %, vorzugsweise 100 %, zu der Sequenz gemäß SEQ ID NO: 14 kodiert,
h) ein abgeschwächtes Polynukleotid (metK), das für eine S-Adenosylmethionin-Synthase (MetK), vorzugsweise für eine S-Adenosylmethionin-Synthase mit einer Sequenzidentität von mindestens 90, 95 oder 98 %, vorzugsweise 100 %, zu der Sequenz gemäß SEQ ID NO: 16 kodiert,
i) ein abgeschwächtes Polynukleotid (metI), das für eine Methionin-Importsystem-Permease (MetI), vorzugsweise für eine Methionin-Importsystem-Permease mit einer Sequenzidentität von mindestens 90, 95 oder 98 %, vorzugsweise 100 %, zu der Sequenz gemäß SEQ ID NO: 18 kodiert,
j) ein abgeschwächtes Polynukleotid (dapA), das für eine 4-Hydroxy-Tetrahydrodipicolinat-Synthase (DapA), vorzugsweise für eine 4-Hydroxy-Tetrahydrodipicolinat-Synthase mit einer Sequenzidentität von mindestens 90, 95 oder 98 %, vorzugsweise 100 %, zu der Sequenz gemäß SEQ ID NO: 20 kodiert,
k) ein überexprimiertes Polynukleotid (CBS), das für eine Cystein-Synthase (CBS), vorzugsweise für eine Cystein-Synthase mit einer Sequenzidentität von mindestens 90, 95 oder 98 %, vorzugsweise 100 %, zu der Sequenz gemäß SEQ ID NO: 22 kodiert,
l) ein abgeschwächtes Polynukleotid, das für ein cg3031-Homolog, vorzugsweise für ein cg3031-Homolog mit einer Sequenzidentität von mindestens 90, 95 oder 98 %, vorzugsweise 100 %, zu der Sequenz gemäß SEQ ID NO: 24 kodiert,
m) ein überexprimiertes Polynukleotid (aecD), das für eine Cystathionin-Betalyase (AecD), vorzugsweise für eine Cystathionin-Betalyase mit einer Sequenzidentität von mindestens 90, 95 oder 98 %, vorzugsweise 100 %, zu der Sequenz gemäß SEQ ID NO: 26 kodiert,
n) ein überexprimiertes Polynukleotid (asd), das für eine Aspartat-semialdehyd-Dehydrogenase (Asd), vorzugsweise für eine Aspartat-semialdehyd-Dehydrogenase mit einer Sequenzidentität von mindestens 90, 95 oder 98 %, vorzugsweise 100 %, zu der Sequenz gemäß SEQ ID NO: 28 kodiert,
o) ein überexprimiertes Polynukleotid (metH), das für eine 5-Methyltetrahydrofolat-Homocysteinmethyltransferase (MetH, EC 2.1.1.13) kodiert,
p) ein überexprimiertes Polynukleotid (brnE), das für die kleinere Untereinheit eines Transporters für verzweigt-kettige Aminosäuren (BrnE), vorzugsweise für eine Untereinheit mit einer Sequenzidentität von mindestens 90, 95 oder 98 %, vorzugsweise 100 %, zu der Sequenz gemäß SEQ ID NO: 30 kodiert,
q) ein überexprimiertes Polynukleotid (brnF), das für die größere Untereinheit eines Transporters für verzweigt-kettige Aminosäuren (BrnF), vorzugsweise für eine Untereinheit mit einer Sequenzidentität von mindestens 90, 95 oder 98 %, vorzugsweise 100 %, zu der Sequenz gemäß SEQ ID NO: 32 kodiert,
r) ein überexprimiertes Polynukleotid (cysE), das für eine Serin-Acetyltransferase (CysE), vorzugsweise für eine Serin-Acetyltransferase mit einer Sequenzidentität von mindestens 90, 95 oder 98 %, vorzugsweise 100 %, zu der Sequenz gemäß SEQ ID NO: 34 kodiert,
s) ein überexprimiertes Polynukleotid (cysK), das für eine Cystein-Synthase (CysK), vorzugsweise für eine Cystein-Synthase mit einer Sequenzidentität von mindestens 90, 95 oder 98 %, vorzugsweise 100 %, zu der Sequenz gemäß SEQ ID NO: 36 kodiert,
t) ein überexprimiertes Polynukleotid (glyA), das für eine Serin-Hydroxymethyltransferase (GlyA), vorzugsweise für eine Serin-Hydroxymethyltransferase mit einer Sequenzidentität von mindestens 90, 95 oder 98 %, vorzugsweise 100 %, zu der Sequenz gemäß SEQ ID NO: 44 kodiert,
u) ein überexprimiertes Polynukleotid (hom), das für eine gegebenenfalls feedback-resistente Homoserin-Dehydrogenase (Hom), vorzugsweise für eine Homoserin-Dehydrogenase mit einer Sequenzidentität von mindestens 90, 95 oder 98 %, vorzugsweise 100 %, zu der Sequenz gemäß SEQ ID NO: 46 kodiert,
v) ein überexprimiertes Polynukleotid (lysC), das für eine gegebenenfalls feedback-resistente Aspartat-Kinase (LysC), vorzugsweise für eine Aspartat-Kinase mit einer Sequenzidentität von mindestens 90, 95 oder 98 %, vorzugsweise 100 %, zu der Sequenz gemäß SEQ ID NO: 54 kodiert,
w) ein überexprimiertes Polynukleotid (metB), das für eine Cystathionin-gamma-Synthase (MetB), vorzugsweise für eine Cystathinonin-gamma-Synthase mit einer Sequenzidentität von mindestens 90, 95 oder 98 %, vorzugsweise 100 %, zu der Sequenz gemäß SEQ ID NO: 56 kodiert,
x) ein überexprimiertes Polynukleotid (metF), das für eine 5,10-Methylentetrahydrofolat-Reduktase (MetF), vorzugsweise für eine 5,10-Methylentetrahydrofolat-Reduktase mit einer Sequenzidentität von mindestens 90, 95 oder 98 %, vorzugsweise 100 %, zu der Sequenz gemäß SEQ ID NO: 58 kodiert,
y) ein überexprimiertes Polynukleotid (metX), das für eine Homoserin-O-Acetyltransferase (MetX), vorzugsweise für eine Homoserin-O-Acetyltransferase mit einer Sequenzidentität von mindestens 90, 95 oder 98 %, vorzugsweise 100 %, zu der Sequenz gemäß SEQ ID NO: 60 kodiert,
z) ein überexprimiertes Polynukleotid (metY), das für eine O-Acetylhomoserin-Lyase (MetY), vorzugsweise für eine O-Acetylhomoserin-Lyase mit einer Sequenzidentität von mindestens 90, 95 oder 98 %, vorzugsweise 100 %, zu der Sequenz gemäß SEQ ID NO: 62 kodiert,
aa) ein überexprimiertes Polynukleotid (pyc), das für eine Pyruvat-Carboxylase (Pyc), vorzugsweise für eine Pyruvat-Carboxylase mit einer Sequenzidentität von mindestens 90, 95 oder 98 %, vorzugsweise 100 %, zu der Sequenz gemäß SEQ ID NO: 64 kodiert,
bb) ein überexprimiertes Polynukleotid (serA), das für eine gegebenenfalls feedback-resistente D-3-Phosphoglycerat-Dehydrogenase (SerA), vorzugsweise für eine D-3-Phosphoglycerat-Dehydrogenase mit einer Sequenzidentität von mindestens 90, 95 oder 98 %, vorzugsweise 100 %, zu der Sequenz gemäß SEQ ID NO: 66 kodiert,
cc) ein überexprimiertes Polynukleotid (serB), das für eine Phosphoserin-Phosphatase (SerB), vorzugsweise für eine Phosphoserin-Phosphatase mit einer Sequenzidentität von mindestens 90, 95 oder 98 %, vorzugsweise 100 %, zu der Sequenz gemäß SEQ ID NO: 68 kodiert,
dd) ein überexprimiertes Polynukleotid (serC), das für eine Phosphoserin-Aminotransferase (SerC), vorzugsweise für eine Phosphoserin-Aminotransferase mit einer Sequenzidentität von mindestens 90, 95 oder 98 %, vorzugsweise 100 %, zu der Sequenz gemäß SEQ ID NO: 70 kodiert,
ee) ein überexprimiertes Polynukleotid (ald), das für eine Alanin-Dehydrogenase (Ald), vorzugsweise für eine Alanin-Dehydrogenase mit einer Sequenzidentität von mindestens 90, 95 oder 98 %, vorzugsweise 100 %, zu der Sequenz gemäß SEQ ID NO: 72 kodiert,
ff) ein überexprimiertes Polynukleotid (cysD), das für die Untereinheit einer Sulfat-Adenylyltransferase (CysD), vorzugsweise für eine Untereinheit mit einer Sequenzidentität von mindestens 90, 95 oder 98 %, vorzugsweise 100 %, zu der Sequenz gemäß SEQ ID NO: 74 kodiert,
gg) ein überexprimiertes Polynukleotid (cysH), das für eine Adenosin-Phosphosulfat-Reduktase (CysH), vorzugsweise für eine Adenosin-Phosphosulfat-Reduktase mit einer Sequenzidentität von mindestens 90, 95 oder 98 %, vorzugsweise 100 %, zu der Sequenz gemäß SEQ ID NO: 76 kodiert,
hh) ein überexprimiertes Polynukleotid (cysl), das für eine Sulfit-Reduktase (Cysl), vorzugsweise für eine Sulfit-Reduktase mit einer Sequenzidentität von mindestens 90, 95 oder 98 %, vorzugsweise 100 %, zu der Sequenz gemäß SEQ ID NO: 78 kodiert,
ii) ein überexprimiertes Polynukleotid (cysJ), das für (CysJ), vorzugsweise für eine mit einer Sequenzidentität von mindestens 90, 95 oder 98 %, vorzugsweise 100 %, zu der Sequenz gemäß SEQ ID NO: 80 kodiert,
jj) ein überexprimiertes Polynukleotid (cysN), das für die Untereinheit einer Sulfat-Adenylyltransferase (CysN), vorzugsweise für eine Untereinheit mit einer Sequenzidentität von mindestens 90, 95 oder 98 %, vorzugsweise 100 %, zu der Sequenz gemäß SEQ ID NO: 82 kodiert,
kk) ein überexprimiertes Polynukleotid (cysY), das für eine Cystathionin-beta-Synthase (CysY), vorzugsweise für eine Cystathionin-beta-Synthase mit einer Sequenzidentität von mindestens 90, 95 oder 98 %, vorzugsweise 100 %, zu der Sequenz gemäß SEQ ID NO: 84 kodiert,
ll) ein überexprimiertes Polynukleotid (cysZ), das für einen putativen Sulfat-Transporter (CysZ), vorzugsweise für einen Sulfat-Transporter mit einer Sequenzidentität von mindestens 90, 95 oder 98 %, vorzugsweise 100 %, zu der Sequenz gemäß SEQ ID NO: 86 kodiert,
mm) ein überexprimiertes Polynukleotid (metE), das für eine 5-Methyltetrahydropteroyl-triglutamat-homocystein-Methyltransferase (MetE), vorzugsweise für ein Protein mit einer Sequenzidentität von mindestens 90, 95 oder 98 %, vorzugsweise 100 %, zu der Sequenz gemäß SEQ ID NO: 88 kodiert,
nn) ein überexprimiertes Polynukleotid (ptH1), das für eine Peptidyl-tRNA-Hydrolase 1 (PtH1), vorzugsweise für eine Peptidyl-tRNA-Hydrolase 1 mit einer Sequenzidentität von mindestens 90, 95 oder 98 %, vorzugsweise 100 %, zu der Sequenz gemäß SEQ ID NO: 90 kodiert,
oo) ein überexprimiertes Polynukleotid (ptH2), das für eine Peptidyl-tRNA-Hydrolase 2 (PtH2), vorzugsweise für eine eine Peptidyl-tRNA-Hydrolase 2 mit einer Sequenzidentität von mindestens 90, 95 oder 98 %, vorzugsweise 100 %, zu der Sequenz gemäß SEQ ID NO: 92 kodiert.

Weiterer Gegenstand der vorliegenden Erfindung ist auch entsprechend ein Verfahren zur Überproduktion einer L-Aminosäure, insbesondere L-Methionin, in welchem ein derartiger Mikroorganismus bzw. ein derartiges Bakterium eingesetzt wird.

In einer weiteren erfindungsgemäß bevorzugten Ausführungsform weisen erfindungsgemäße Mikroorganismen oder Bakterien, insbesondere erfindungsgemäße Corynebakterien, vor allem erfindungsgemäße Corynebakterien der Art C. humireducens oder C. glutamicum, insbesondere erfindungsgemäße L-Valin-Überproduktionsstämme, neben einem erfindungsgemäßen, vorzugsweise überexprimierten, Glycin spaltenden System bzw. der für dieses kodierenden Polynukleotide, mindestens eines, vorzugsweise mindestens 2 oder 3, besonders bevorzugt mindestens 4 oder 5, der folgenden Merkmale auf:
a) ein überexprimiertes Polynukleotid (ilvA-Gen), das für eine Threonindehydratase (IlvA, EC 4.3.1.19), vorzugsweise für eine Threonindehydratase mit einer Sequenzidentität von mindestens 90, 95 oder 98 %, vorzugsweise 100 %, zu der Sequenz gemäß SEQ ID NO: 106, kodiert,
b) ein überexprimiertes Polynukleotid (ilvB-Gen), das für die Untereinheit einer Acetolactat-Synthase (IlvB), vorzugsweise für die Untereinheit einer Acetolactat-Synthase mit einer Sequenzidentität von mindestens 90, 95 oder 98 %, vorzugsweise 100 %, zu der Sequenz gemäß SEQ ID NO: 98, kodiert,
c) ein überexprimiertes Polynukleotid (ilvN-Gen), das für die gegebenenfalls feedback-resistente Untereinheit einer Acetolactat-Synthase (IlvN) kodiert,
d) ein überexprimiertes Polynukleotid (ilvC-Gen), das für eine Isomeroreduktase (IlvC, EC 1.1.1.86), vorzugsweise für eine Isomeroreduktase mit einer Sequenzidentität von mindestens 90, 95 oder 98 %, vorzugsweise 100 %, zu der Sequenz gemäß SEQ ID NO: 100, kodiert,
e) ein überexprimiertes Polynukleotid (ilvD-Gen), das für eine Dihydroxy-acid Dehydratase (IlvD, EC 4.2.1.9), vorzugsweise für eine Dihydroxy-acid Dehydratase mit einer Sequenzidentität von mindestens 90, 95 oder 98 %, vorzugsweise 100 %, zu der Sequenz gemäß SEQ ID NO: 102, kodiert,
f) ein überexprimiertes Polynukleotid (ilvE-Gen), das für eine Transaminase (IlvE, EC 2.6.1.42), vorzugsweise für eine Transaminase mit einer Sequenzidentität von mindestens 90, 95 oder 98 %, vorzugsweise 100 %, zu der Sequenz gemäß SEQ ID NO: 104, kodiert,
g) ein überexprimiertes Polynukleotid (ilvH-Gen), das für eine Acetolactat-Synthase (IlvH, EC 2.2.1.6), vorzugsweise für eine Acetolactat-Synthase mit einer Sequenzidentität von mindestens 90, 95 oder 98 %, vorzugsweise 100 %, zu der Sequenz gemäß SEQ ID NO: 122, kodiert,
h) ein abgeschwächtes Polynukleotid (thrB-Gen), das für eine Homoserinkinase (ThrB, EC 2.7.1.39), vorzugsweise für eine Homoserinkinase mit einer Sequenzidentität von mindestens 90, 95 oder 98 %, vorzugsweise 100 %, zu der Sequenz gemäß SEQ ID NO: 4, kodiert,
i) ein abgeschwächtes Polynukleotid (thrC-Gen), das für eine Threonin-Synthase (ThrC, EC 4.2.3.1), vorzugsweise für eine Threonin-Synthase mit einer Sequenzidentität von mindestens 90, 95 oder 98 %, vorzugsweise 100 %, zu der Sequenz gemäß SEQ ID NO: 108, kodiert,
j) ein überexprimiertes Polynukleotid (hom-Gen), das für eine gegebenenfalls feedback-resistente Homoserin-Dehydrogenase (Hom, EC 1.2.1.11), vorzugsweise für eine Homoserin-Dehydrogenase mit einer Sequenzidentität von mindestens 90, 95 oder 98 %, vorzugsweise 100 %, zu der Sequenz gemäß SEQ ID NO: 46, kodiert,
k) ein abgeschwächtes Polynukleotid (leuA-Gen), das für eine gegebenenfalls feedback-resistente Isopropylmalat-Synthase (LeuA, EC 2.3.3.13), vorzugsweise für eine Isopropylmalat-Synthase mit einer Sequenzidentität von mindestens 90, 95 oder 98 %, vorzugsweise 100 %, zu der Sequenz gemäß SEQ ID NO: 110, kodiert,
l) ein abgeschwächtes Polynukleotide (leuB-Gen), das für eine Isopropylmalat-Dehydrogenase (LeuB, EC 1.1.1.85), vorzugsweise für eine Isopropylmalat-Dehydrogenase mit einer Sequenzidentität von mindestens 90, 95 oder 98 %, vorzugsweise 100 %, zu der Sequenz gemäß SEQ ID NO: 112, kodiert,
m) abgeschwächtes Polynukleotide (leuCD-Gene), die für die Untereinheiten einer Isopropylmalat-Isomerase (LeuCD, EC 4.2.1.33), vorzugsweise für die Untereinheiten einer Isopropylmalat-Isomerase mit Sequenzidentitäten von mindestens 90, 95 oder 98 %, vorzugsweise 100 %, zu den Sequenzen gemäß SEQ ID NO: 114 und SEQ ID NO: 116, kodieren,
n) ein überexprimiertes Polynukleotid (panB-Gen), das für eine 3-Methyl-2-Oxobutanoat-Hydroxymethyltransferase (PanB, EC 2.1.2.11), vorzugsweise für eine 3-Methyl-2-Oxobutanoat-Hydroxymethyltransferase mit einer Sequenzidentität von mindestens 90, 95 oder 98 %, vorzugsweise 100 %, zu der Sequenz gemäß SEQ ID NO: 118, kodiert,
o) ein überexprimiertes Polynukleotid (panC-Gen), das für eine Pantothenat-Synthase (PanC, EC 6.3.2.1), vorzugsweise für eine Pantothenat-Synthase mit einer Sequenzidentität von mindestens 90, 95 oder 98 %, vorzugsweise 100 %, zu der Sequenz gemäß SEQ ID NO: 120, kodiert.

Weiterer Gegenstand der vorliegenden Erfindung ist entsprechend auch ein Verfahren zur Überproduktion einer L-Aminosäure, insbesondere L-Valin, in welchem ein derartiger Mikroorganismus bzw. ein derartiges Bakterium eingesetzt wird.

In einer weiteren erfindungsgemäß bevorzugten Ausführungsform weisen erfindungsgemäße Mikroorganismen oder Bakterien, insbesondere erfindungsgemäße Corynebakterien, vor allem erfindungsgemäße Corynebakterien der Art C. humireducens oder C. glutamicum, insbesondere erfindungsgemäße L-Glutamat-Überproduktionsstämme, neben einem erfindungsgemäßen, vorzugsweise überexprimierten, Glycin spaltenden System bzw. der für dieses kodierenden Polynukleotide, mindestens eines, vorzugsweise mindestens zwei oder drei, besonders bevorzugt mindestens vier oder fünf, der folgenden Merkmale auf:
a) ein überexprimiertes Polynukleotid (gdh), das für eine Glutamat-Dehydrogenase (Gdh), vorzugsweise für eine Glutamat-Dehydrogenase mit einer Sequenzidentität von mindestens 90, 95 oder 98 %, vorzugsweise 100 %, zu der Sequenz gemäß SEQ ID NO: 124, kodiert,
b) ein überexprimiertes Polynukleotid, das für eine Glutamin-Synthetase (Glutamin-Synthetase 1), vorzugsweise für eine Glutamin-Synthetase mit einer Sequenzidentität von mindestens 90, 95 oder 98 %, vorzugsweise 100 %, zu der Sequenz gemäß SEQ ID NO: 126, kodiert,
c) ein überexprimiertes Polynukleotid, das für eine Glutamin-Synthetase (Glutamin-Synthetase 2), vorzugsweise für eine Glutamin-Synthetase mit einer Sequenzidentität von mindestens 90, 95 oder 98 %, vorzugsweise 100 %, zu der Sequenz gemäß SEQ ID NO: 128, kodiert,
d) ein überexprimiertes Polynukleotid, das für eine Glutamat-Synthase, vorzugsweise für eine Glutamat-Synthase mit einer Sequenzidentität von mindestens 90, 95 oder 98 %, vorzugsweise 100 %, zu der Sequenz gemäß SEQ ID NO: 130, kodiert,
e) ein überexprimiertes Polynukleotid, das für eine Isocitrat-Dehydrogenase, vorzugsweise für eine Isocitrat-Dehydrogenase mit einer Sequenzidentität von mindestens 90, 95 oder 98 %, vorzugsweise 100 %, zu der Sequenz gemäß SEQ ID NO: 132, kodiert,
f) ein überexprimiertes Polynukleotid, das für eine Aconitat-Hydrase, vorzugsweise für eine Aconitat-Hydrase mit einer Sequenzidentität von mindestens 90, 95 oder 98 %, vorzugsweise 100 %, zu der Sequenz gemäß SEQ ID NO: 134, kodiert,
g) ein überexprimiertes Polynukleotid, das für eine Citrat-Synthase, vorzugsweise für eine Citrat-Synthase mit einer Sequenzidentität von mindestens 90, 95 oder 98 %, vorzugsweise 100 %, zu der Sequenz gemäß SEQ ID NO: 136, kodiert,
h) ein überexprimiertes Polynukleotid (pepC), das für eine Aminopeptidase C (PepC), vorzugsweise für eine Aminopeptidase C mit einer Sequenzidentität von mindestens 90, 95 oder 98 %, vorzugsweise 100 %, zu der Sequenz gemäß SEQ ID NO: 138, kodiert,
i) ein überexprimiertes Polynukleotid, das für eine Pyruvat-Dehydrogenase, vorzugsweise für eine Pyruvat-Dehydrogenase mit einer Sequenzidentität von mindestens 90, 95 oder 98 %, vorzugsweise 100 %, zu der Sequenz gemäß SEQ ID NO: 140, kodiert,
j) ein überexprimiertes Polynukleotid, das für eine Pyruvat-Kinase (Pyruvat-Kinase 1), vorzugsweise für eine Pyruvat-Kinase mit einer Sequenzidentität von mindestens 90, 95 oder 98 %, vorzugsweise 100 %, zu der Sequenz gemäß SEQ ID NO: 142, kodiert,
k) ein überexprimiertes Polynukleotid, das für eine Pyruvat-Kinase (Pyruvat-Kinase 2), vorzugsweise für eine Pyruvat-Kinase mit einer Sequenzidentität von mindestens 90, 95 oder 98 %, vorzugsweise 100 %, zu der Sequenz gemäß SEQ ID NO: 144, kodiert,
l) ein überexprimiertes Polynukleotid, das für eine Enolase, vorzugsweise für eine Enolase mit einer Sequenzidentität von mindestens 90, 95 oder 98 %, vorzugsweise 100 %, zu der Sequenz gemäß SEQ ID NO: 146 kodiert,
m) ein überexprimiertes Polynukleotid (gpmA), das für eine 2,3-Bisphosphoglycerat-abhängige Phosphoglycerat-Mutase (GpmA), vorzugsweise für eine Phosphoglycerat-Mutase mit einer Sequenzidentität von mindestens 90, 95 oder 98 %, vorzugsweise 100 %, zu der Sequenz gemäß SEQ ID NO: 148 kodiert,
n) ein überexprimiertes Polynukleotid (pgk), das für eine Phosphoglycerat-Kinase (Pgk), vorzugsweise für eine Phosphoglycerat-Kinase mit einer Sequenzidentität von mindestens 90, 95 oder 98 %, vorzugsweise 100 %, zu der Sequenz gemäß SEQ ID NO: 150 kodiert,
o) ein überexprimiertes Polynukleotid, das für eine Glycerinaldehyd-3-phosphat-Dehydrogenase (Glycerin-3-phosphat-Dehydrogenase 1), vorzugsweise für eine Glycerinaldehyd-3-phosphat-Dehydrogenase mit einer Sequenzidentität von mindestens 90, 95 oder 98 %, vorzugsweise 100 %, zu der Sequenz gemäß SEQ ID NO: 152 kodiert,
p) ein überexprimiertes Polynukleotid, das für eine Glycerinaldehyd-3-phosphat-Dehydrogenase (Glycerin-3-phosphat-Dehydrogenase 2), vorzugsweise für eine Glycerinaldehyd-3-phosphat-Dehydrogenase mit einer Sequenzidentität von mindestens 90, 95 oder 98 %, vorzugsweise 100 %, zu der Sequenz gemäß SEQ ID NO: 154 kodiert,
q) ein überexprimiertes Polynukleotid (tpiA), das für eine Triosephosphat-Isomerase (TpiA), vorzugsweise für eine Triosephosphat-Isomerase mit einer Sequenzidentität von mindestens 90, 95 oder 98 %, vorzugsweise 100 %, zu der Sequenz gemäß SEQ ID NO: 156 kodiert,
r) ein überexprimiertes Polynukleotid, das für eine Fructosebisphosphataldolase, vorzugsweise für eine Fructosebisphosphataldolase mit einer Sequenzidentität von mindestens 90, 95 oder 98 %, vorzugsweise 100 %, zu der Sequenz gemäß SEQ ID NO: 158 kodiert,
s) ein überexprimiertes Polynukleotid, das für eine 1-Phosphofructokinase, vorzugsweise für eine 1-Phosphofructokinase mit einer Sequenzidentität von mindestens 90, 95 oder 98 %, vorzugsweise 100 %, zu der Sequenz gemäß SEQ ID NO: 160 kodiert,
t) ein überexprimiertes Polynukleotid, das für eine 6-Phosphofructokinase, vorzugsweise für eine 6-Phosphofructokinase mit einer Sequenzidentität von mindestens 90, 95 oder 98 %, vorzugsweise 100 %, zu der Sequenz gemäß SEQ ID NO: 162 kodiert,
u) ein überexprimiertes Polynukleotid (pgi), das für eine Glucose-6-phosphat-lsomerase, vorzugsweise für eine Glucose-6-phosphat-lsomerase mit einer Sequenzidentität von mindestens 90, 95 oder 98 %, vorzugsweise 100 %, zu der Sequenz gemäß SEQ ID NO: 6 kodiert,
v) abgeschwächte Polynukleotide (sucCD), die für die Untereinheiten einer Succinyl-CoA-Ligase (SucCD, EC 6.2.1.5), vorzugsweise für die Untereinheiten einer Succinyl-CoA-Ligase mit Sequenzidentitäten von mindestens 90, 95 oder 98 %, vorzugsweise 100 %, zu den Sequenzen gemäß SEQ ID NO: 198 bzw. SEQ ID NO: 200 kodieren.

Weiterer Gegenstand der vorliegenden Erfindung ist entsprechend auch ein Verfahren zur Überproduktion einer L-Aminosäure, insbesondere L-Glutamat, in welchem ein derartiger Mikroorganismus bzw. ein derartiges Bakterium eingesetzt wird.

In einer weiteren erfindungsgemäß bevorzugten Ausführungsform weisen erfindungsgemäße Mikroorganismen oder Bakterien, insbesondere erfindungsgemäße Corynebakterien, vor allem erfindungsgemäße Corynebakterien der Art C. humireducens oder C. glutamicum, insbesondere erfindungsgemäße L-Alanin-Überproduktionsstämme, neben einem erfindungsgemäßen, vorzugsweise überexprimierten, Glycin spaltenden System bzw. der für dieses kodierenden Polynukleotide, mindestens eines, vorzugsweise mindestens zwei oder drei, besonders bevorzugt mindestens vier oder fünf, der folgenden Merkmale auf:
a) ein überexprimiertes Polynukleotid (alaD), das für eine Alanin-Dehydrogenase (AlaD), vorzugsweise für eine Alanin-Dehydrogenase aus Corynebakterien kodiert,
b) ein überexprimiertes Polynukleotid (gapA), das für eine Glycerinaldehyd-3-phosphat-Dehydrogenase (GapA), vorzugsweise für eine Glycerinaldehyd-3-phosphat-Dehydrogenase aus Corynebakterien kodiert,
c) ein ausgeschaltetes oder abgeschwächtes Polynukleotid (ldhA), das für eine L-Lactat-Dehydrogenase (LdhA), vorzugsweise für eine L-Lactat-Dehydrogenase aus Corynebakterien kodiert,
d) ein ausgeschaltetes oder abgeschwächtes Polynukleotid (ppc), das für eine Phosphoenolpyruvat-Carboxylase (Ppc), vorzugsweise für eine Phosphoenolpyruvat-Carboxylase aus Corynebakterien kodiert,
e) ein ausgeschaltetes oder abgeschwächtes Polynukleotid (alr), das für eine Alanin-Racemase (Alr), vorzugsweise für eine Alanin-Racemase aus Corynebakterien kodiert.

Weiterer Gegenstand der vorliegenden Erfindung ist entsprechend auch ein Verfahren zur Überproduktion einer L-Aminosäure, insbesondere L-Alanin, in welchem ein derartiger Mikroorganismus bzw. ein derartiges Bakterium eingesetzt wird.

In einer weiteren erfindungsgemäß bevorzugten Ausführungsform weisen erfindungsgemäße Mikroorganismen oder Bakterien, insbesondere erfindungsgemäße Corynebakterien, vor allem erfindungsgemäße Corynebakterien der Art C. humireducens oder C. glutamicum, insbesondere erfindungsgemäße L-Lysin-Überproduktionsstämme, neben einem erfindungsgemäßen, vorzugsweise überexprimierten, Glycin spaltenden System bzw. der für dieses kodierenden Polynukleotide, mindestens eines, vorzugsweise mindestens 2 oder 3, besonders bevorzugt mindestens 4 oder 5, der folgenden Merkmale auf:
a) ein überexprimiertes Polynukleotid (dapA-Gen), das für eine Dihydrodipicolinat-Synthase (DapA, EC 4.2.1.52), vorzugsweise für eine Dihydrodipicolinat-Synthase mit einer Sequenzidentität von mindestens 90, 95 oder 98 %, vorzugsweise 100 %, zu der Sequenz gemäß SEQ ID NO: 20, kodiert,
b) ein überexprimierter Polynukleotid (lysC), das für eine vorzugsweise feedback-resistente Aspartat-Kinase (LysC, EC 2.7.2.4), vorzugsweise für eine Aspartat-Kinase mit einer Sequenzidentität von mindestens 90, 95 oder 98 %, vorzugsweise 100 %, zu der Sequenz gemäß SEQ ID NO: 54 kodiert,
c) ein überexprimiertes Polynukleotid (ddh), das für eine Diaminopimelat-Dehydrogenase (Ddh, EC 1.4.1.16), vorzugsweise für eine Diaminopimelat-Dehydrogenase mit einer Sequenzidentität von mindestens 90, 95 oder 98 %, vorzugsweise 100 %, zu der Sequenz gemäß SEQ ID NO: 202 kodiert,
d) ein überexprimiertes Polynukleotid (asd-Gen), das für eine Aspartat-Semialdehyd-Dehydrogenase (Asd, EC 1.2.1.11), vorzugsweise für eine eine Aspartat-Semialdehyd-Dehydrogenase mit einer Sequenzidentität von mindestens 90, 95 oder 98 %, vorzugsweise 100 %, zu der Sequenz gemäß SEQ ID NO: 28, kodiert,
e) ein überexprimiertes Polynukleotid (lysA-Gen), das für eine Diaminopimelat-Decarboxylase (LysA, EC 4.1.1.20), vorzugsweise für eine Diaminopimelat-Decarboxylase mit einer Sequenzidentität von mindestens 90, 95 oder 98 %, vorzugsweise 100 %, zu der Sequenz gemäß SEQ ID NO: 164, kodiert,
f) ein überexprimiertes Polynukleotid (aat-Gen), das für eine Aspartat-Aminotransferase (AaT, EC 2.6.1.1), vorzugsweise für eine Aspartat-Aminotransferase mit einer Sequenzidentität von mindestens 90, 95 oder 98 %, vorzugsweise 100 %, zu der Sequenz gemäß SEQ ID NO: 166, kodiert,
g) ein überexprimiertes Polynukleotid (lysE-Gen), das für einen L-Lysin-Exporter (LysE, Lysin-Efflux-Permease), vorzugsweise für einen L-Lysin-Exporter mit einer Sequenzidentität von mindestens 90, 95 oder 98 %, vorzugsweise 100 %, zu der Sequenz gemäß SEQ ID NO: 168, kodiert,
h) ein überexprimiertes Polynukleotid (pyc-Gen), das für eine Pyruvat-Carboxylase (Pyc, EC 6.4.1.1), vorzugsweise für eine Pyruvat-Carboxylase mit einer Sequenzidentität von mindestens 90, 95 oder 98 %, vorzugsweise 100 %, zu der Sequenz gemäß SEQ ID NO: 64, kodiert,
i) ein überexprimiertes Polynukleotid (dapF-Gen), das für eine Diaminopimelat-Epimerase (DapF, EC 5.1.1.7) kodiert,
j) ein überexprimiertes Polynukleotid (dapB-Gen), das für eine Dihydropicolinat-Reduktase (DapB, EC 1.3.1.26), vorzugsweise für eine Dihydropicolinat-Reduktase mit einer Sequenzidentität von mindestens 90, 95 oder 98 %, vorzugsweise 100 %, zu der Sequenz gemäß SEQ ID NO: 172, kodiert,
k) ein überexprimiertes Polynukleotid, das für eine Glucose-6-phosphat-Dehydrogenase (EC 1.1.1.49), vorzugsweise für eine Glucose-6-phosphat-Dehydrogenase mit einer Sequenzidentität von mindestens 90, 95 oder 98 %, vorzugsweise 100 %, zu der Sequenz gemäß SEQ ID NO: 174, kodiert,
l) ein überexprimiertes Polynukleotid (zwf-Gen), das für die Zwf-Untereinheit einer Glucose-6-phosphat-Dehydrogenase (Zwf, EC 1.1.1.49), vorzugsweise für eine Zwf-Untereinheit mit einer Sequenzidentität von mindestens 90, 95 oder 98 %, vorzugsweise 100 %, zu der Sequenz gemäß SEQ ID NO: 188, kodiert,
m) ein überexprimiertes Polynukleotid (opcA-Gen), das für die OpcA-Untereinheit einer Glucose-6-phosphat-Dehydrogenase (OpcA, EC 1.1.1.49), vorzugsweise für eine OpcA-Untereinheit mit einer Sequenzidentität von mindestens 90, 95 oder 98 %, vorzugsweise 100 %, zu der Sequenz gemäß SEQ ID NO: 190, kodiert,
n) ein überexprimiertes Polynukleotid (gnd-Gen), das für eine Phosphogluconsäure-Dehydrogenase (Gnd, EC 1.1.1.44), vorzugsweise für eine Phosphogluconsäure-Dehydrogenase mit einer Sequenzidentität von mindestens 90, 95 oder 98 %, vorzugsweise 100 %, zu der Sequenz gemäß SEQ ID NO: 176, kodiert,
o) ein ausgeschaltetes oder abgeschwächtes Polynukleotid (mqo), das für eine Malat-Chinon-Oxidoreduktase (Mqo, EC 1.1.99.16), vorzugsweise für eine Malat-Chinon-Oxidoreduktase mit einer Sequenzidentität von mindestens 90, 95 oder 98 %, vorzugsweise 100 %, zu der Sequenz gemäß SEQ ID NO: 178, kodiert,
p) ein ausgeschaltetes oder abgeschwächtes Polynukleotid (aceE), das für die E1p-Untereinheit eines Pyruvat-Dehydrogenase-Komplexes (AceE, EC 1.2.4.1), vorzugsweise für eine E1p-Untereinheit mit einer Sequenzidentität von mindestens 90, 95 oder 98 %, vorzugsweise 100 %, zu der Sequenz gemäß SEQ ID NO: 180, kodiert,
q) ein ausgeschaltetes oder abgeschwächtes Polynukleotid (gltA), das für eine Citrat-Synthase (GltA, EC 4.1.3.7), vorzugsweise für eine Citrat-Synthase mit einer Sequenzidentität von mindestens 90, 95 oder 98 %, vorzugsweise 100 %, zu der Sequenz gemäß SEQ ID NO: 182, kodiert,
r) ein ausgeschaltetes oder abgeschwächtes Polynukleotid (mdh), das für eine Malat-Dehydrogenase (Mdh, EC 1.1.1.37), vorzugsweise für eine Malat-Dehydrogenase mit einer Sequenzidentität von mindestens 90, 95 oder 98 %, vorzugsweise 100 %, zu der Sequenz gemäß SEQ ID NO: 184, kodiert,
s) ein ausgeschaltetes oder abgeschwächtes Polynukleotid (murE), das für eine UDP-N-acetylmuramoylalanyl-D-glutamat-2,6-diaminopimelat-Ligase (MurE, EC 6.3.2.13), vorzugsweise für ein Enzym mit einer Sequenzidentität von mindestens 90, 95 oder 98 %, vorzugsweise 100 %, zu der Sequenz gemäß SEQ ID NO: 186, kodiert.

Weiterer Gegenstand der vorliegenden Erfindung ist entsprechend auch ein Verfahren zur Überproduktion einer L-Aminosäure, insbesondere L-Lysin, in welchem ein derartiger Mikroorganismus bzw. ein derartiges Bakterium eingesetzt wird.

Die zuvor genannten, in erfindungsgemäßen Verfahren eingesetzten bzw. einzusetzenden Polynukleotide und Polypeptide stammen vorzugsweise aus Corynebakterien, insbesondere aus C. glutamicum oder C. humireducens, besonders bevorzugt aus C. humireducens.

Unter "Überexpression" ist erfindungsgemäß allgemein eine Erhöhung der intrazellulären Konzentration oder Aktivität einer Ribonukleinsäure, eines Proteins (Polypeptids) oder eines Enzyms, die durch eine entsprechende DNA kodiert werden, in einem Mikroorganismus im Vergleich zum Ausgangsstamm (Elternstamm) oder Wildtypstamm zu verstehen. Unter einem Ausgangsstamm (Elternstamm) versteht man den Stamm, an dem die zur Überexpression führende Maßnahme durchgeführt wurde.

Die Erhöhung der Konzentration oder Aktivität lässt sich beispielsweise dadurch erzielen, dass man die Kopienzahl der entsprechenden kodierenden Polynukleotide chromosomal oder extrachromosomal um mindestens eine Kopie erhöht.

Eine weit verbreitete Methode zur Erhöhung der Kopienzahl besteht darin, dass man das entsprechende kodierende Polynukleotid in einen Vektor, bevorzugt ein Plasmid, einbaut, der von einem Mikroorganismuns, insbesondere einem coryneformen Bakterium, repliziert wird. Weiterhin kann man als Vektoren Transposons, Insertionselemente (IS-Elemente) oder Phagen einsetzen. Im Stand der Technik ist eine Fülle geeigneter Vektoren beschrieben.

Eine andere verbreitete Methode zur Erzielung einer Überexpression ist das Verfahren der chromosomalen Genamplifikation. Bei dieser Methode wird mindestens eine zusätzliche Kopie des interessierenden Polynukleotids in das Chromosom eines coryneformen Bakteriums eingefügt. Derartige Amplifikationsverfahren sind beispielsweise in der WO 03/014330 oder WO 03/040373 beschrieben.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in der Fig. 1 des Übersichtsartikel von Patek et al. (Journal of Biotechnology 104(1-3), 311-323 (2003)) und in zusammenfassenden Darstellungen wie dem "Handbook of Corynebacterium glutamicum" (Eds.: Lothar Eggeling und Michael Bott, CRC Press, Boca Raton, US (2005)) oder dem Buch "Corynebacteria, Genomics and Molecular Biology" (Ed.: Andreas Burkovski, Caister Academic Press, Norfolk, UK (2008)) beschrieben. In gleicher Weise können die von Vasicova et al (Journal of Bacteriology 181, 6188-6191 (1999)) beschriebenen Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des betreffenden Gens, typischerweise im Abstand von ungefähr 1 - 500 Nukleobasen vom Startkodon, eingefügt werden.

Durch die Maßnahme der Überexpression wird die Aktivität oder Konzentration des entsprechenden Polypeptids vorzugsweise um mindestens 10%, 25%, 50%, 75%, 100%, 150%, 200%, 300%, 400% oder 500%, maximal bis vorzugsweise 1000% oder 2000%, bezogen auf die Aktivität oder Konzentration des Polypeptids im Stamm vor der zur Überexpression führenden Maßnahme, erhöht.

Die Konzentration eines Proteins kann über 1- und 2-dimensionale Proteingelauftrennung und anschließende optische Identifizierung der Proteinkonzentration mit entsprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden. Die Aktivität kann mit Hilfe eines geeigneten Enzymtest bestimmt werden.

Der Begriff "Abschwächung" bezeichnet erfindungsgemäß die Verringerung der intrazellulären Konzentration oder Aktivität einer Ribonukleinsäure, eines Proteins (Polypeptids) oder eines Enzyms, die durch eine entsprechende DNA kodiert werden, in einem Mikroorganismus, im Vergleich zum Ausgangsstamm (Elternstamm) oder Wildtypstamm. Als Ausgangsstamm (Elternstamm) wird der Stamm bezeichnet, an dem die Maßnahme der Abschwächung durchgeführt wurde.

Die Abschwächung kann erzielt werden, indem die Expression eines Polypeptids, beispielsweise durch Verwendung eines schwachen Promotors, verringert wird oder indem ein Allel verwendet wird, das für ein Polypeptid mit einer niedrigeren Aktivität kodiert und gegebenenfalls diese Maßnahmen kombiniert. Die Abschwächung kann auch dadurch erreicht werden, indem die Expression des Polypeptids vollständig unterbunden wird, beispielsweise dadurch, dass das kodierende Gen ausgeschaltet wird.

Durch die Maßnahme der Abschwächung wird die Aktivität oder Konzentration des entsprechenden Polypeptids vorzugsweise um mindestens 10%, 25%, 50% oder 75%, maximal um 100%, bezogen auf die Aktivität oder Konzentration des Polypeptids im Stamm vor der zur Abschwächung führenden Maßnahme, reduziert. In einer bevorzugten Ausführungsform besteht die Abschwächung in der vollständigen Ausschaltung der Expression des betreffenden Polypeptids.

Unter feedback-resistenten Enzymen versteht man im Zusammenhang mit der Aminosäureproduktion generell Enzyme, die im Vergleich zur Wildform eine geringere Empfindlichkeit gegenüber der Hemmung durch die produzierte L-Aminosäure und/oder Analoga davon aufweisen.

So versteht man insbesondere unter einer feedback-resistenten Aspartatkinase (LysC^{FBR}) eine Aspartatkinase, die im Vergleich zur Wildform eine geringere Empfindlichkeit gegenüber der Hemmung durch Mischungen von Lysin und Threonin oder Mischungen von AEC (Aminoethylcystein) und Threonin oder Lysin alleine oder AEC alleine aufweisen. Für die Lysin-Produktion werden entsprechend Stämme, die solche feedbackresistenten bzw. desensibilisierten Aspartatkinasen enthalten, bevorzugt eingesetzt.

Literaturbekannt sind beispielsweise folgende feedback-resistenten Aspartatkinasen aus C. glutamicum: A279T, A279V, S301F, S301Y, T308I, T311I, R320G, G345D, S381F. Bzgl. feedback-resistenten Aspartatkinasen aus C. glutamicum wird weiterhin auf folgende Veröffentlichungen verwiesen: JP1993184366-A, JP1994062866-A, JP1994261766-A, JP1997070291-A, JP1997322774-A, JP1998165180-A, JP1998215883-A, US5688671-A, EP0387527, WO00/63388, US3732144, JP6261766, Jetten et al. (1995; Applied Microbiology Biotechnology 43: 76-82). Feedback-resistente Aspartatkinasen aus C. glutamicum sind in der NCBI-GenBank unter folgenden Zugangsnummern hinterlegt: E05108, E06825, E06826, E06827, E08177, E08178, E08179, E08180, E08181, E08182, E12770, E14514, E16352, E16745, E16746, I74588, I74589, I74590, I74591, I74592, I74593, I74594, I74595, I74596, I74597, X57226, L16848, L27125.

Bevorzugt werden erfindungsgemäß folgende feedback-resistenten Aspartatkinasen aus C. humireducens eingesetzt: D274Y, A279E, S301Y, T308I, T311I, G359D.

Ebenso werden bei der Threonin-Produktion bevorzugt Stämme eingesetzt, die entsprechend eine feedback-resistente Homoserin-Dehydrogenase (Hom^{FBR}) enthalten.

Ebenso werden bei der Isoleucin-Produktion und Valin-Produktion bevorzugt Stämme eingesetzt, die entsprechend eine feedback-resistente Acetolactat-Synthase enthalten.

Ebenso werden bei der Leucin-Produktion bevorzugt Stämme eingesetzt, die entsprechend eine feedback-resistente Isopropylmalat-Synthase (LeuA^{FBR}) enthalten.

Ebenso werden bei der Prolin-Produktion bevorzugt Stämme eingesetzt, die entsprechend eine feedback-resistente Glutamat-5-Kinase (ProB^{FBR}) enthalten.

Ebenso werden bei der Arginin-Produktion bevorzugt Stämme eingesetzt, die entsprechend eine feedback-resistente Ornithin-Carbamoyltransferase (ArgF^{FBR}) enthalten.

Ebenso werden bei der Serin-Produktion bevorzugt Stämme eingesetzt, die entsprechend eine feedback-resistente D-3-Phosphoglycerat-Dehydrogenase (SerA^{FBR}) enthalten.

Ebenso werden bei der Methionin-Produktion bevorzugt Stämme eingesetzt, die entsprechend eine feedback-resistente D-3-Phosphoglycerat-Dehydrogenase (SerA^{FBR}) und/oder feedback-resistente Pyruvat-Carboxylasen (Pyc^{FBR}) enthalten.

Ebenso werden bei der Tryptophan-Produktion bevorzugt Stämme eingesetzt, die entsprechend eine feedback-resistente Phospho-2-dehydro-3-Deoxyheptonat-Aldolase (AroG^{FBR} oder AroH^{FBR}) enthalten.

Hinsichtlich weiterer bevorzugter Eigenschaften des erfindungsgemäß einzusetzenden L-Aminosäuren überproduzierenden C. humireducens-Stamms wird auf die oben zitierte Veröffentlichung von Wu et al. (2011) sowie die weiteren oben genannten Veröffentlichungen verwiesen.

Erfindungsgemäße Mikroorganismen, insbesondere Bakterien der Gattung Corynebacterium, können kontinuierlich - wie beispielsweise in der WO 05/021772 beschrieben- oder diskontinuierlich im Batch-Verfahren (Satzkultivierung bzw. Satzverfahren) oder im Fed-Batch- (Zulaufverfahren) oder Repeated-Fed-Batch Verfahren (repetitives Zulaufverfahren) zum Zwecke der Produktion von L-Lysin kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlenhydrate wie z.B. Glucose, Saccharose, Lactose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnussöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten L-Aminosäure, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Bakterien kommt es zu einer Anreicherung (Akkumulation) der L-Aminosäure im Fermentationsmedium und/oder in den Bakterienzellen.

Beispiele für geeignete Fermentationsmedien finden sich unter anderem in den Patentschriften 5,770,409, US 5,840,551 und US 5,990,350 oder US 5,275,940.

Die Analyse von L-Aminosäuren zur Bestimmung der Konzentration zu einem oder mehreren Zeitpunkt(en) im Verlauf der Fermentation kann durch Trennung der L-Aminosäuren mittels lonenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur lonenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeits-chromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Gegenstand der Erfindung ist dementsprechend auch ein Verfahren zur Herstellung einer L-Aminosäure, dadurch gekennzeichnet, dass man folgende Schritte durchführt:
a) Fermentation der erfindungsgemäßen Mikroorganismen, insbesondere coryneformen Bakterien, bevorzugt der Gattung Corynebacterium, besonders bevorzugt der Art Corynebacterium glutamicum oder Corynebacterium humireducens, in einem geeignetem Nährmedium, und
b) Akkumulation der L-Aminosäure in dem Nährmedium und/oder in den Zellen der genannten Bakterien.

Anschließend erfolgt die Bereitstellung bzw. Herstellung oder Gewinnung eines L-Aminosäure haltigen Produktes in flüssiger oder fester Form.

Durch die Maßnahmen der Fermentation erhält man eine Fermentationsbrühe, welche die betreffende L-Aminosäure enthält.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Vermehrung des Mikroorganismus und eine Bildung der gewünschten L-Aminosäure sicherstellen.

Bei Abschluss der Fermentation enthält die entstandene Fermentationsbrühe dementsprechend
a) die infolge der Vermehrung der Zellen des Mikroorganismus entstandene Biomasse (Zellmasse) des Mikroorganismus,
b) das im Laufe der Fermentation gebildete L-Aminosäure,
c) die im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Mikroorganismen neben der gewünschten L-Aminosäure erzeugt und gegebenenfalls ausgeschieden werden. Hierzu gehören auch Zucker wie zum Beispiel Trehalose.

Die Fermentationsbrühe wird dem Kulturgefäß beziehungsweise dem Fermentationsbehälter entnommen, gegebenenfalls gesammelt, und dazu verwendet, ein L-Aminosäure haltiges Produkt in flüssiger oder fester Form bereitzustellen. Hierfür wird auch der Ausdruck "Gewinnen des L-Aminosäure haltigen Produktes" verwendet. Im einfachsten Fall stellt die L-Aminosäure-haltige Fermentationsbrühe selbst das gewonnene Produkt dar.

Durch eine oder mehrere der Maßnahmen ausgewählt aus der Gruppe
a) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung des Wassers,
b) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung der Biomasse, wobei diese gegebenenfalls vor der Entfernung inaktiviert wird,
c) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der im Laufe der Fermentation gebildeten organischen Nebenprodukte, und
d) teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der durch die Fermentation nicht verbrauchten Bestandteile des eingesetzten Fermentationsmediums beziehungsweise der Einsatzstoffe,
aus der Fermentationsbrühe erzielt man eine Konzentrierung bzw. Reinigung der L-Aminosäure. Auf diese Weise werden Produkte isoliert, die einen gewünschten Gehalt an L-Aminosäure aufweisen.

Die teilweise (> 0% bis < 80%) bis vollständige (100%) oder nahezu vollständige (≥ 80% bis < 100%) Entfernung des Wassers (Maßnahme a)) wird auch als Trocknung bezeichnet.

Durch vollständige oder nahezu vollständige Entfernung des Wassers, der Biomasse, der organischen Nebenprodukte und der nicht verbrauchten Bestandteile des eingesetzten Fermentationsmediums gelangt man zu reinen ((≥ 80 Gew.-%, ≥ 90 Gew.-%) oder hochreinen (≥ 95 Gew.-%, ≥ 97 Gew.-%, ≥ 99% Gew.-%) Produktformen der L-Aminosäure. Für die Maßnahmen gemäß a), b), c) oder d) sind im Stand der Technik eine Fülle von technischen Anleitungen verfügbar.

### Ausführungsbeispiele

### Beispiel 1: Alanin- und Valin-Produktion durch C. humireducens

Zur Überprüfung der Alanin- und Valin-Produktion wurde der Typstamm von C. *humireducens* (DSM 45392) im Schüttelkolbenansatz kultiviert. Dazu wurde der C. *humireducens* Stamm in 10 ml BHI-Flüssigmedium (Brain-Heart-Infusion; Merck) (37 g/l H₂O) als Vorkultur bei 37°C mit 200 rpm 24 h lang inkubiert. Anschließend wurden 10 ml Schüttelkolbenmedium auf eine OD₆₆₀ von 0,2 inokuliert und bei 37°C mit 200 rpm, 48 h lang kultiviert. Zur Herstellung dieses Mediums wurden 20 g Ammoniumsulfat, 0,4 g MgSO₄*7H₂O, 0,6 g KH₂PO₄ und 10 g Hefeextrakt in 750 ml H₂O gelöst. Der pH-Wert der Lösung wurde mit 20% NH₄OH auf 7,8 eingestellt und die Lösung anschließend autoklaviert. Anschließend wurden 4 ml einer Vitaminlösung (pH 7 mit NH₄OH), bestehend aus 0,25 g/l Thiamin, 50 mg/l Cyanocobalamin, 25 mg/l Biotin und 1,25 g/l Pyridoxin, hinzugefügt. Des Weiteren wurden 140 ml einer sterilfiltrierten 50%igen Glukoselösung und 50 g trockenautoklaviertes CaCO₃ hinzugefügt und das Medium anschließend auf einen Liter aufgefüllt.
Nach der Kultivierung wurde jeweils vom Überstand von vier parallelen Kulturen eine HPLC-Analyse zur Bestimmung des Gehaltes an Alanin, Glycin und Valin mit einer Nachweisgrenze von ≥0,01 g/l durchgeführt.

Der Typstamm von C. *humireducens* produziert nach 48 h Kultivierung in Schüttelkolbenmedium bei 37°C, 200 rpm im Schüttelkolbenmaßstab rund 0,81 g/l Alanin (Netto-Ausbeute: 0,011 g_{Alanin}/g_{Glukose}) und 1,6 g/l Valin (Netto-Ausbeute: 0,022 gᵥₐₗᵢₙ/g_{Glukose}) (Tab. 1). Glycin fiel nur in geringer Menge als Nebenprodukt an.

**Tab. 1: Analytikwerte eines Schüttelkolbenversuches mit dem Typstamm von C. humireducens. Aufgeführt sind die gemessenen Werte nach der Kultivierung mit Zellen und die des leeren Mediums.**

| | **Alanin (g/l)** | **Valin (g/l)** |
|---|---|---|
| | | |
| **C. humireducens** | 1,27 | 1,9 |
| | | |
| **Leer-Medium ohne Zellen** | 0,46 | 0,3 |

### Beispiel 3: Glutamat-Leistungstest

Für den L-Glutamat-Leistungstest wurde der Typstamm von C. *humireducens* (DSM 45392) im Schüttelkolbenansatz kultiviert. Dazu wurde der C. *humireducens* Stamm in 10 ml BHI-Flüssigmedium (Brain-Heart-Infusion; Merck) (37 g/l H₂O) als Vorkultur bei 37°C mit 200 rpm 24 h lang inkubiert. Anschließend wurden 10 ml Schüttelkolbenmedium auf eine OD₆₆₀ von 0,2 inokuliert und bei 37°C mit 200 rpm, 48 h lang kultiviert. Zur Herstellung dieses Mediums wurden 20 g Ammoniumsulfat, 0,4 g MgSO₄*7H₂O, 0,6 g KH₂PO₄ und 10 g Hefeextrakt in 750 ml H₂O gelöst. Der pH-Wert der Lösung wurde mit 20% NH₄OH auf 7,8 eingestellt und die Lösung anschließend autoklaviert. Anschließend wurden 4 ml einer Vitaminlösung (pH 7 mit NH₄OH), bestehend aus 0,25 g/l Thiamin, 50 mg/l Cyanocobalamin, 25 mg/l Biotin und 1,25 g/l Pyridoxin hinzugefügt. Des Weiteren wurden 140 ml einer sterilfiltrierten 50%igen Glukoselösung und 50 g trockenautoklaviertes CaCO₃ hinzugefügt. Daraufhin kamen noch 5 ml einer 400 mM sterilfiltierten Threonin-Stammlösung hinzu und das Medium wurde anschließend auf einen Liter aufgefüllt.

Nach der Kultivierung wurde jeweils vom Überstand von vier parallelen Kulturen eine HPLC-Analyse zur Bestimmung des Glutamatgehaltes mit einer Nachweisgrenze von ≥0,01 g/l durchgeführt.

Der Typstamm von C. *humireducens* produzierte nach 48 h Kultivierung in Schüttelkolbenmedium bei 37°C, 200 rpm im Schüttelkolbenmaßstab 1,8 (+/-0,6) g/l L-Glutamat. Die Ausgangskonzentration an L- Glutamat im Medium lag bei 0,78 (+/-0,1) g/l. Glycin fiel nur in geringer Menge als Nebenprodukt an.

## Patentansprüche

1. Glycin spaltendes System umfassend die Enzyme GcvP, GcvT und GcvH, **dadurch gekennzeichnet, dass** es mindestens eines der folgenden Polypeptide umfasst:
a) ein Enzym GcvP mit einer Sequenzidentität von mindestens 80 % zu der Sequenz gemäß SEQ ID NO: 40,
b) ein Enzym GcvT mit einer Sequenzidentität von mindestens 80 % zu der Sequenz gemäß SEQ ID NO: 42,
c) ein Enzym GcvH mit einer Sequenzidentität von mindestens 80 % zu der Sequenz gemäß SEQ ID NO: 38.

2. Glycin spaltendes System nach Anspruch 1, **dadurch gekennzeichnet, dass** es mindestens zwei, vorzugsweise alle drei, der zuvor genannten Polypeptide umfasst.

3. Glycin spaltendes System nach Anspruch 2, **dadurch gekennzeichnet, dass** es folgende Polypeptide umfasst:
a) ein Enzym GcvP mit einer Sequenzidentität von mindestens 95 oder 98 % zu der Sequenz gemäß SEQ ID NO: 40,
b) ein Enzym GcvT mit einer Sequenzidentität von mindestens 95 oder 98 % zu der Sequenz gemäß SEQ ID NO: 42,
c) ein Enzym GcvH mit einer Sequenzidentität von mindestens 95 oder 98 % zu der Sequenz gemäß SEQ ID NO: 38.

4. Glycin spaltendes System nach einem der vorhergehenden Ansprüche, **dadurch gekennzeichnet, dass** es mindestens ein weiteres Polypeptid umfasst, ausgewählt aus der Gruppe bestehend aus:
a) ein Enzym LipA mit einer Sequenzidentität von mindestens 80 % zu der Sequenz gemäß SEQ ID NO: 48,
b) ein Enzym LipB mit einer Sequenzidentität von mindestens 80 % zu der Sequenz gemäß SEQ ID NO: 50,
c) ein Enzym Lpd mit einer Sequenzidentität von mindestens 80 % zu der Sequenz gemäß SEQ ID NO: 52,
d) ein Enzym LplA mit einer Sequenzidentität von mindestens 80 % zu der Sequenz gemäß SEQ ID NO: 94,
e) ein Enzym GcvL mit einer Sequenzidentität von mindestens 80 % zu der Sequenz gemäß SEQ ID NO: 96.

5. Polypeptide, die Enzyme eines Glycin spaltenden Systems darstellen, ausgewählt aus der Gruppe bestehend aus:
a) ein Enzym GcvP mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 40,
b) ein Enzym GcvT mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 42,
c) ein Enzym GcvH mit einer Sequenzidentität von mindestens 80 %, vorzugsweise mindestens 85 oder 90 %, insbesondere mindestens 92, 94, 96 oder 98 %, vor allem 100 %, zu der Sequenz gemäß SEQ ID NO: 38.

6. Polynukleotide, die für Enzyme eines Glycin spaltenden Systems kodieren, ausgewählt aus der Gruppe bestehend aus:
a) ein Polynukleotid (gcvP), das für das Enzym GcvP kodiert und eine Sequenzidentität von mindestens 70 oder 75 %, vorzugsweise mindestens 80 oder 85 %, insbesondere mindestens 90 oder 95 %, vor allem mindestens 98 oder 100 %, zu der Sequenz von Position 301 bis 3162 gemäß SEQ ID NO: 39 aufweist und/oder unter stringenten Bedingungen mit einem Polynukleotid hybridisiert, dessen Sequenz komplementär zu der Sequenz von Position 301 bis 3162 gemäß SEQ ID NO: 39 ist;
b) ein Polynukleotid (gcvT), das für das Enzym GcvT kodiert und eine Sequenzidentität
von mindestens 70 oder 75 %, vorzugsweise mindestens 80 oder 85 %, insbesondere mindestens 90 oder 95 %, vor allem mindestens 98 oder 100 %, zu der Sequenz von Position 301 bis 1404 gemäß SEQ ID NO: 41 aufweist und/oder unter stringenten Bedingungen mit einem Polynukleotid hybridisiert, dessen Sequenz komplementär zu der Sequenz von Position 301 bis 1404 gemäß SEQ ID NO: 41 ist;
c) ein Polynukleotid (gcvH), das für das Enzym GcvH kodiert und eine Sequenzidentität von mindestens 70 oder 75 %, vorzugsweise mindestens 80 oder 85 %, insbesondere mindestens 90 oder 95 %, vor allem mindestens 98 oder 100 %, zu der Sequenz von Position 301 bis 699 gemäß SEQ ID NO: 37 aufweist und/oder unter stringenten Bedingungen mit einem Polynukleotid hybridisiert, dessen Sequenz komplementär zu der Sequenz von Position 301 bis 699 gemäß SEQ ID NO: 37 ist.

7. Vektor, **dadurch gekennzeichnet, dass** er mindestens ein Polynukleotid, vorzugsweise mindestens zwei oder drei Polynukleotide, gemäß Anspruch 6 umfasst.

8. Rekombinanter Mikroorganisums, **dadurch gekennzeichnet, dass** er mindestens ein Glycin spaltendes System nach einem der Ansprüche 1 bis 4 und/oder für ein solches Glycin spaltendes System kodierende Polynukleotide und/oder mindestens ein Polypeptid nach Anspruch 5 und/oder ein für ein solches Polypeptid kodierendes Polynukleotid bzw. für solche Polypeptide kodierende Polynukleotide und/oder mindestens ein Polynukleotid nach Anspruch 6 und/oder einen Vektor nach Anspruch 7 umfasst.

9. Rekombinanter Mikroorganismus nach Anspruch 8, **dadurch gekennzeichnet, dass** das Glycin spaltende System und/oder für ein solches kodierende Polynukleotide in überexprimierter Form vorliegen.

10. Rekombinanter Mikroorganismus nach Anspruch 8 oder 9, **dadurch gekennzeichnet, dass** es sich um einen L-Methionin-Überproduktionsstamm handelt und dass er mindestens eines, vorzugsweise mindestens zwei oder drei, besonders bevorzugt mindestens vier oder fünf, der folgenden Merkmale aufweist:
a) ein abgeschwächtes Polynukleotid (mcbR), das für eine DNA-Bindungsdomäne vom Typ HTH tetR (McbR), vorzugsweise für eine DNA-Bindungsdomäne mit einer Sequenzidentität von mindestens 90, 95 oder 98 %, vorzugsweise 100 %, zu der Sequenz gemäß SEQ ID NO: 2, kodiert,
b) ein abgeschwächtes Polynukleotid (thrB-Gen), das für eine Homoserinkinase (ThrB, EC 2.7.1.39), vorzugsweise für eine Homoserinkinase mit einer Sequenzidentität von mindestens 90, 95 oder 98 %, vorzugsweise 100 %, zu der Sequenz gemäß SEQ ID NO: 4, kodiert,
c) ein abgeschwächtes Polynukleotid (pgi), das für eine Glucose-6-phosphat-Isomerase (Pgi, EC 5.3.1.9), vorzugsweise für eine Glucose-6-phosphat-Isomerase mit einer Sequenzidentität von mindestens 90, 95 oder 98 %, vorzugsweise 100 %, zu der Sequenz gemäß SEQ ID NO: 6 kodiert,
d) ein abgeschwächtes Polynukleotid (pck), das für eine Phosphoenolpyruvat-Carboxykinase (Pck, EC 4.1.1.32), vorzugsweise für eine Phosphoenolpyruvat-Carboxykinase mit einer Sequenzidentität von mindestens 90, 95 oder 98 %, vorzugsweise 100 %, zu der Sequenz gemäß SEQ ID NO: 8 kodiert,
e) ein abgeschwächtes Polynukleotid (metQ), das für ein D-Methionin bindendes Lipoprotein (MetQ), vorzugsweise für ein D-Methionin bindendes Lipoprotein mit einer Sequenzidentität von mindestens 90, 95 oder 98 %, vorzugsweise 100 %, zu der Sequenz gemäß SEQ ID NO: 10 kodiert,
f) ein abgeschwächtes Polynukleotid (metP), das für einen Methionin-Transporter (MetP), vorzugsweise für einen Methionin-Transporter mit einer Sequenzidentität von mindestens 90, 95 oder 98 %, vorzugsweise 100 %, zu der Sequenz gemäß SEQ ID NO: 12 kodiert,
g) ein abgeschwächtes Polynukleotid (metN), das für einen ATP-abhängigen Methionin-Transporter (MetN), vorzugsweise für einen ATP-abhängigen Methionin-Transporter mit einer Sequenzidentität von mindestens 90, 95 oder 98 %, vorzugsweise 100 %, zu der Sequenz gemäß SEQ ID NO: 14 kodiert,
h) ein abgeschwächtes Polynukleotid (metK), das für eine S-Adenosylmethionin-Synthase (MetK), vorzugsweise für eine S-Adenosylmethionin-Synthase mit einer Sequenzidentität von mindestens 90, 95 oder 98 %, vorzugsweise 100 %, zu der Sequenz gemäß SEQ ID NO: 16 kodiert,
i) ein abgeschwächtes Polynukleotid (metI), das für eine Methionin-Importsystem-Permease (MetI), vorzugsweise für eine Methionin-Importsystem-Permease mit einer Sequenzidentität von mindestens 90, 95 oder 98 %, vorzugsweise 100 %, zu der Sequenz gemäß SEQ ID NO: 18 kodiert,
j) ein abgeschwächtes Polynukleotid (dapA), das für eine 4-Hydroxy-Tetrahydrodipicolinat-Synthase (DapA), vorzugsweise für eine 4-Hydroxy-Tetrahydrodipicolinat-Synthase mit einer Sequenzidentität von mindestens 90, 95 oder 98 %, vorzugsweise 100 %, zu der Sequenz gemäß SEQ ID NO: 20 kodiert,
k) ein überexprimiertes Polynukleotid (CBS), das für eine Cystein-Synthase (CBS), vorzugsweise für eine Cystein-Synthase mit einer Sequenzidentität von mindestens 90, 95 oder 98 %, vorzugsweise 100 %, zu der Sequenz gemäß SEQ ID NO: 22 kodiert,
l) ein abgeschwächtes Polynukleotid, das für ein cg3031-Homolog, vorzugsweise für ein cg3031-Homolog mit einer Sequenzidentität von mindestens 90, 95 oder 98 %, vorzugsweise 100 %, zu der Sequenz gemäß SEQ ID NO: 24 kodiert,
m) ein überexprimiertes Polynukleotid (aecD), das für eine Cystathionin-Betalyase (AecD), vorzugsweise für eine Cystathionin-Betalyase mit einer Sequenzidentität von mindestens 90, 95 oder 98 %, vorzugsweise 100 %, zu der Sequenz gemäß SEQ ID NO: 26 kodiert,
n) ein überexprimiertes Polynukleotid (asd), das für eine Aspartat-semialdehyd-Dehydrogenase (Asd), vorzugsweise für eine Aspartat-semialdehyd-Dehydrogenase mit einer Sequenzidentität von mindestens 90, 95 oder 98 %, vorzugsweise 100 %, zu der Sequenz gemäß SEQ ID NO: 28 kodiert,
o) ein überexprimiertes Polynukleotid (metH), das für eine 5-Methyltetrahydrofolat-Homocysteinmethyltransferase (MetH, EC 2.1.1.13) kodiert,
p) ein überexprimiertes Polynukleotid (brnE), das für die kleinere Untereinheit eines Transporters für verzweigt-kettige Aminosäuren (BrnE), vorzugsweise für eine Untereinheit mit einer Sequenzidentität von mindestens 90, 95 oder 98 %, vorzugsweise 100 %, zu der Sequenz gemäß SEQ ID NO: 30 kodiert,
q) ein überexprimiertes Polynukleotid (brnF), das für die größere Untereinheit eines Transporters für verzweigt-kettige Aminosäuren (BrnF), vorzugsweise für eine Untereinheit mit einer Sequenzidentität von mindestens 90, 95 oder 98 %, vorzugsweise 100 %, zu der Sequenz gemäß SEQ ID NO: 32 kodiert,
r) ein überexprimiertes Polynukleotid (cysE), das für eine Serin-Acetyltransferase (CysE), vorzugsweise für eine Serin-Acetyltransferase mit einer Sequenzidentität von mindestens 90, 95 oder 98 %, vorzugsweise 100 %, zu der Sequenz gemäß SEQ ID NO: 34 kodiert,
s) ein überexprimiertes Polynukleotid (cysK), das für eine Cystein-Synthase (CysK), vorzugsweise für eine Cystein-Synthase mit einer Sequenzidentität von mindestens 90, 95 oder 98 %, vorzugsweise 100 %, zu der Sequenz gemäß SEQ ID NO: 36 kodiert,
t) ein überexprimiertes Polynukleotid (glyA), das für eine Serin-Hydroxymethyltransferase (GlyA), vorzugsweise für eine Serin-Hydroxymethyltransferase mit einer Sequenzidentität von mindestens 90, 95 oder 98 %, vorzugsweise 100 %, zu der Sequenz gemäß SEQ ID NO: 44 kodiert,
u) ein überexprimiertes Polynukleotid (hom), das für eine gegebenenfalls feedback-resistente Homoserin-Dehydrogenase (Hom), vorzugsweise für eine Homoserin-Dehydrogenase mit einer Sequenzidentität von mindestens 90, 95 oder 98 %, vorzugsweise 100 %, zu der Sequenz gemäß SEQ ID NO: 46 kodiert,
v) ein überexprimiertes Polynukleotid (lysC), das für eine gegebenenfalls feedback-resistente Aspartat-Kinase (LysC), vorzugsweise für eine Aspartat-Kinase mit einer Sequenzidentität von mindestens 90, 95 oder 98 %, vorzugsweise 100 %, zu der Sequenz gemäß SEQ ID NO: 54 kodiert,
w) ein überexprimiertes Polynukleotid (metB), das für eine Cystathionin-gamma-Synthase (MetB), vorzugsweise für eine Cystathinonin-gamma-Synthase mit einer Sequenzidentität von mindestens 90, 95 oder 98 %, vorzugsweise 100 %, zu der Sequenz gemäß SEQ ID NO: 56 kodiert,
x) ein überexprimiertes Polynukleotid (metF), das für eine 5,10-Methylentetrahydrofolat-Reduktase (MetF), vorzugsweise für eine 5,10-Methylentetrahydrofolat-Reduktase mit einer Sequenzidentität von mindestens 90, 95 oder 98 %, vorzugsweise 100 %, zu der Sequenz gemäß SEQ ID NO: 58 kodiert,
y) ein überexprimiertes Polynukleotid (metX), das für eine Homoserin-O-Acetyltransferase (MetX), vorzugsweise für eine Homoserin-O-Acetyltransferase mit einer Sequenzidentität von mindestens 90, 95 oder 98 %, vorzugsweise 100 %, zu der Sequenz gemäß SEQ ID NO: 60 kodiert,
z) ein überexprimiertes Polynukleotid (metY), das für eine O-Acetylhomoserin-Lyase (MetY), vorzugsweise für eine O-Acetylhomoserin-Lyase mit einer Sequenzidentität von mindestens 90, 95 oder 98 %, vorzugsweise 100 %, zu der Sequenz gemäß SEQ ID NO: 62 kodiert,
aa) ein überexprimiertes Polynukleotid (pyc), das für eine Pyruvat-Carboxylase (Pyc), vorzugsweise für eine Pyruvat-Carboxylase mit einer Sequenzidentität von mindestens 90, 95 oder 98 %, vorzugsweise 100 %, zu der Sequenz gemäß SEQ ID NO: 64 kodiert,
bb) ein überexprimiertes Polynukleotid (serA), das für eine gegebenenfalls feedback-resistente D-3-Phosphoglycerat-Dehydrogenase (SerA), vorzugsweise für eine D-3-Phosphoglycerat-Dehydrogenase mit einer Sequenzidentität von mindestens 90, 95 oder 98 %, vorzugsweise 100 %, zu der Sequenz gemäß SEQ ID NO: 66 kodiert,
cc) ein überexprimiertes Polynukleotid (serB), das für eine Phosphoserin-Phosphatase (SerB), vorzugsweise für eine Phosphoserin-Phosphatase mit einer Sequenzidentität von mindestens 90, 95 oder 98 %, vorzugsweise 100 %, zu der Sequenz gemäß SEQ ID NO: 68 kodiert,
dd) ein überexprimiertes Polynukleotid (serC), das für eine Phosphoserin-Aminotransferase (SerC), vorzugsweise für eine Phosphoserin-Aminotransferase mit einer Sequenzidentität von mindestens 90, 95 oder 98 %, vorzugsweise 100 %, zu der Sequenz gemäß SEQ ID NO: 70 kodiert,
ee) ein überexprimiertes Polynukleotid (ald), das für eine Alanin-Dehydrogenase (Ald), vorzugsweise für eine Alanin-Dehydrogenase mit einer Sequenzidentität von mindestens 90, 95 oder 98 %, vorzugsweise 100 %, zu der Sequenz gemäß SEQ ID NO: 72 kodiert,
ff) ein überexprimiertes Polynukleotid (cysD), das für die Untereinheit einer Sulfat-Adenylyltransferase (CysD), vorzugsweise für eine Untereinheit mit einer Sequenzidentität von mindestens 90, 95 oder 98 %, vorzugsweise 100 %, zu der Sequenz gemäß SEQ ID NO: 74 kodiert,
gg) ein überexprimiertes Polynukleotid (cysH), das für eine Adenosin-Phosphosulfat-Reduktase (CysH), vorzugsweise für eine Adenosin-Phosphosulfat-Reduktase mit einer Sequenzidentität von mindestens 90, 95 oder 98 %, vorzugsweise 100 %, zu der Sequenz gemäß SEQ ID NO: 76 kodiert,
hh) ein überexprimiertes Polynukleotid (cysl), das für eine Sulfit-Reduktase (Cysl), vorzugsweise für eine Sulfit-Reduktase mit einer Sequenzidentität von mindestens 90, 95 oder 98 %, vorzugsweise 100 %, zu der Sequenz gemäß SEQ ID NO: 78 kodiert,
ii) ein überexprimiertes Polynukleotid (cysJ), das für (CysJ), vorzugsweise für eine mit einer Sequenzidentität von mindestens 90, 95 oder 98 %, vorzugsweise 100 %, zu der Sequenz gemäß SEQ ID NO: 80 kodiert,
jj) ein überexprimiertes Polynukleotid (cysN), das für die Untereinheit einer Sulfat-Adenylyltransferase (CysN), vorzugsweise für eine Untereinheit mit einer Sequenzidentität von mindestens 90, 95 oder 98 %, vorzugsweise 100 %, zu der Sequenz gemäß SEQ ID NO: 82 kodiert,
kk) ein überexprimiertes Polynukleotid (cysY), das für eine Cystathionin-beta-Synthase (CysY), vorzugsweise für eine Cystathionin-beta-Synthase mit einer Sequenzidentität von mindestens 90, 95 oder 98 %, vorzugsweise 100 %, zu der Sequenz gemäß SEQ ID NO: 84 kodiert,
ll) ein überexprimiertes Polynukleotid (cysZ), das für einen putativen Sulfat-Transporter (CysZ), vorzugsweise für einen Sulfat-Transporter mit einer Sequenzidentität von mindestens 90, 95 oder 98 %, vorzugsweise 100 %, zu der Sequenz gemäß SEQ ID NO: 86 kodiert,
mm) ein überexprimiertes Polynukleotid (metE), das für eine 5-Methyltetrahydropteroyl-triglutamat-homocystein-Methyltransferase (MetE), vorzugsweise für ein Protein mit einer Sequenzidentität von mindestens 90, 95 oder 98 %, vorzugsweise 100 %, zu der Sequenz gemäß SEQ ID NO: 88 kodiert,
nn) ein überexprimiertes Polynukleotid (ptH1), das für eine Peptidyl-tRNA-Hydrolase 1 (PtH1), vorzugsweise für eine Peptidyl-tRNA-Hydrolase 1 mit einer Sequenzidentität von mindestens 90, 95 oder 98 %, vorzugsweise 100 %, zu der Sequenz gemäß SEQ ID NO: 90 kodiert,
oo) ein überexprimiertes Polynukleotid (ptH2), das für eine Peptidyl-tRNA-Hydrolase 2 (PtH2), vorzugsweise für eine eine Peptidyl-tRNA-Hydrolase 2 mit einer Sequenzidentität von mindestens 90, 95 oder 98 %, vorzugsweise 100 %, zu der Sequenz gemäß SEQ ID NO: 92 kodiert.

11. Rekombinanter Mikroorganismus nach einem der Ansprüche 8 bis 10, **dadurch gekennzeichnet, dass** es sich um ein coryneformes Bakterium, insbesondere um ein Corynebacterium, vorzugsweise um C. glutamicum oder C. humireducens, handelt.

12. Verfahren zur Überproduktion einer L-Aminosäure, **dadurch gekennzeichnet, dass** in diesem Verfahren ein Glycin spaltendes System nach einem der Ansprüche 1 bis 4 und/oder mindestens ein Polypeptid nach Anspruch 5 und/oder mindestens ein Polynukleotid nach Anspruch 6 und/oder ein Vektor nach Anspruch 7 und/oder ein rekombinanter Mikroorganismus nach einem der Ansprüche 8 bis 11 eingesetzt wird.

13. Verfahren nach vorhergehendem Anspruch, **dadurch gekennzeichnet, dass** die überproduzierte L-Aminosäure ausgewählt ist aus L-Alanin, L-Valin, L-Aminosäuren der Glutamat-Familie, insbesondere L-Glutamat, L-Glutamin, L-Prolin und L-Arginin, sowie L-Aminosäuren der Aspartat-Familie, insbesondere L-Aspartat, L-Asparagin, L-Methionin, L-Lysin, L-Isoleucin und L-Threonin.

14. Verfahren nach Anspruch 13, **dadurch gekennzeichnet, dass** es sich bei der überproduzierten L-Aminosäure um L-Methionin handelt.

15. Verfahren nach einem der vorhergehenden Ansprüche, **dadurch gekennzeichnet, dass** nur geringe Mengen an Glycin als Nebenprodukt anfallen.

## Claims

1. Glycine cleavage system comprising the enzymes GcvP, GcvT and GcvH, **characterized in that** said system comprises at least one of the following polypeptides:
a) an enzyme GcvP having a sequence identity of at least 80% to the sequence according to SEQ ID NO: 40,
b) an enzyme GcvT having a sequence identity of at least 80% to the sequence according to SEQ ID NO: 42,
c) an enzyme GcvH having a sequence identity of at least 80% to the sequence according to SEQ ID NO: 38.

2. Glycine cleavage system according to Claim 1, **characterized in that** said system comprises two, preferably all three of the polypeptides mentioned above.

3. Glycine cleavage system according to Claim 2, **characterized in that** said system comprises the following polypeptides:
a) an enzyme GcvP having a sequence identity of at least 95 or 98% to the sequence according to SEQ ID NO: 40,
b) an enzyme GcvT having a sequence identity of at least 95 or 98% to the sequence according to SEQ ID NO: 42,
c) an enzyme GcvH having a sequence identity of at least 95 or 98% to the sequence according to SEQ ID NO: 38.

4. Glycine cleavage system according to any of the preceding claims, **characterized in that** said system comprises at least one further polypeptide selected from the group consisting of:
a) an enzyme LipA having a sequence identity of at least 80% to the sequence according to SEQ ID NO: 48,
b) an enzyme LipB having a sequence identity of at least 80% to the sequence according to SEQ ID NO: 50,
c) an enzyme Lpd having a sequence identity of at least 80% to the sequence according to SEQ ID NO: 52,
d) an enzyme LplA having a sequence identity of at least 80% to the sequence according to SEQ ID NO: 94,
e) an enzyme GcvL having a sequence identity of at least 80% to the sequence according to SEQ ID NO: 96.

5. Polypeptides, which constitute enzymes of a glycine cleavage system, selected from the group consisting of:
a) an enzyme GcvP having a sequence identity of at least 80%, preferably at least 85 or 90%, particularly at least 92, 94, 96 or 98%, especially 100%, to the sequence according to SEQ ID NO: 40,
b) an enzyme GcvT having a sequence identity of at least 80%, preferably at least 85 or 90%, particularly at least 92, 94, 96 or 98%, especially 100%, to the sequence according to SEQ ID NO: 42,
c) an enzyme GcvH having a sequence identity of at least 80%, preferably at least 85 or 90%, particularly at least 92, 94, 96 or 98%, especially 100%, to the sequence according to SEQ ID NO: 38.

6. Polynucleotides, which code for enzymes of a glycine cleavage system, selected from the group consisting of:
a) a polynucleotide (gcvP), which codes for the enzyme GcvP and has a sequence identity of at least 70 or 75%, preferably at least 80 or 85%, particularly at least 90 or 95%, especially at least 98 or 100%, to the sequence of position 301 to 3162 according to SEQ ID NO: 39 and/or hybridizes under stringent conditions with a polynucleotide of which the sequence is complementary to the sequence of position 301 to 3162 according to SEQ ID NO: 39;
b) a polynucleotide (gcvT), which codes for the enzyme GcvT and has a sequence identity of at least 70 or 75%, preferably at least 80 or 85%, particularly at least 90 or 95%, especially at least 98 or 100%, to the sequence of position 301 to 1404 according to SEQ ID NO: 41 and/or hybridizes under stringent conditions with a polynucleotide of which the sequence is complementary to the sequence of position 301 to 1404 according to SEQ ID NO: 41;
c) a polynucleotide (gcvH), which codes for the enzyme GcvH and has a sequence identity of at least 70 or 75%, preferably at least 80 or 85%, particularly at least 90 or 95%, especially at least 98 or 100%, to the sequence of position 301 to 699 according to SEQ ID NO: 37 and/or hybridizes under stringent conditions with a polynucleotide of which the sequence is complementary to the sequence of position 301 to 699 according to SEQ ID NO: 37.

7. Vector, **characterized in that** said vector comprises at least one polynucleotide, preferably at least two or three polynucleotides, according to Claim 6.

8. Recombinant microorganism, **characterized in that** said microorganism comprises at least one glycine cleavage system according to any of Claims 1 to 4 and/or polynucleotides coding for such a glycine cleavage system and/or at least one polypeptide according to Claim 5 and/or a polynucleotide coding for such a polypeptide or polynucleotides coding for such polypeptides and/or at least one polynucleotide according to Claim 6 and/or a vector according to Claim 7.

9. Recombinant microorganism according to Claim 8, **characterized in that** the glycine cleavage system and/or polynucleotides coding for such a system are present in overexpressed form.

10. Recombinant microorganism according to Claim 8 or 9, **characterized in that** said microorganism is an L-methionine-overproducing strain having at least one, preferably at least two or three, particularly preferably at least four or five, of the following features:
a) an attenuated polynucleotide (mcbR), which codes for a DNA binding domain of the type HTH tetR (McbR), preferably for a DNA binding domain having a sequence identity of at least 90, 95 or 98%, preferably 100%, to the sequence according to SEQ ID NO: 2,
b) an attenuated polynucleotide (thrB gene), which codes for a homoserine kinase (ThrB, EC 2.7.1.39), preferably for a homoserine kinase having a sequence identity of at least 90, 95 or 98%, preferably 100%, to the sequence according to SEQ ID NO: 4,
c) an attenuated polynucleotide (pgi), which codes for a glucose-6-phosphate isomerase (Pgi, EC 5.3.1.9), preferably for a glucose-6-phosphate isomerase having a sequence identity of at least 90, 95 or 98%, preferably 100%, to the sequence according to SEQ ID NO: 6,
d) an attenuated polynucleotide (pck), which codes for a phosphoenolpyruvate carboxykinase (Pck, EC 4.1.1.32), preferably for a phosphoenolpyruvate carboxykinase having a sequence identity of at least 90, 95 or 98%, preferably 100%, to the sequence according to SEQ ID NO: 8,
e) an attenuated polynucleotide (metQ), which codes for a D-methionine-binding lipoprotein (MetQ), preferably for a D-methionine-binding lipoprotein having a sequence identity of at least 90, 95 or 98%, preferably 100%, to the sequence according to SEQ ID NO: 10,
f) an attenuated polynucleotide (metP), which codes for a methionine transporter (MetP), preferably for a methionine transporter having a sequence identity of at least 90, 95 or 98%, preferably 100%, to the sequence according to SEQ ID NO: 12,
g) an attenuated polynucleotide (metN), which codes for an ATP-dependent methionine transporter (MetN), preferably for an ATP-dependent methionine transporter having a sequence identity of at least 90, 95 or 98%, preferably 100%, to the sequence according to SEQ ID NO: 14,
h) an attenuated polynucleotide (metK), which codes for an S-adenosylmethionine synthase (MetK), preferably for an S-adenosylmethionine synthase having a sequence identity of at least 90, 95 or 98%, preferably 100%, to the sequence according to SEQ ID NO: 16,
i) an attenuated polynucleotide (metI), which codes for a methionine import system permease (MetI), preferably for a methionine import system permease having a sequence identity of at least 90, 95 or 98%, preferably 100%, to the sequence according to SEQ ID NO: 18,
j) an attenuated polynucleotide (dapA), which codes for a 4-hydroxy-tetrahydrodipicolinate synthase (DapA), preferably for a 4-hydroxy-tetrahydrodipicolinate synthase having a sequence identity of at least 90, 95 or 98%, preferably 100%, to the sequence according to SEQ ID NO: 20,
k) an overexpressed polynucleotide (CBS), which codes for a cysteine synthase (CBS), preferably for a cysteine synthase having a sequence identity of at least 90, 95 or 98%, preferably 100%, to the sequence according to SEQ ID NO: 22,
l) an attenuated polynucleotide, which codes for a cg3031 homologue, preferably for a cg3031 homologue having a sequence identity of at least 90, 95 or 98%, preferably 100%, to the sequence according to SEQ ID NO: 24,
m) an overexpressed polynucleotide (aecD), which codes for a cystathionine beta-lyase (AecD), preferably for a cystathionine beta-lyase having a sequence identity of at least 90, 95 or 98%, preferably 100%, to the sequence according to SEQ ID NO: 26,
n) an overexpressed polynucleotide (asd), which codes for an aspartate semialdehyde dehydrogenase (Asd), preferably for an aspartate semialdehyde dehydrogenase having a sequence identity of at least 90, 95 or 98%, preferably 100%, to the sequence according to SEQ ID NO: 28,
o) an overexpressed polynucleotide (metH), which codes for a 5-methyltetrahydrofolate homocysteine methyltransferase (MetH, EC 2.1.1.13),
p) an overexpressed polynucleotide (brnE), which codes for the smaller subunit of a transporter for branched-chain amino acids (BrnE), preferably for a subunit having a sequence identity of at least 90, 95 or 98%, preferably 100%, to the sequence according to SEQ ID NO: 30,
q) an overexpressed polynucleotide (brnF), which codes for the larger subunit of a transporter for branched-chain amino acids (BrnF), preferably for a subunit having a sequence identity of at least 90, 95 or 98%, preferably 100%, to the sequence according to SEQ ID NO: 32,
r) an overexpressed polynucleotide (cysE), which codes for a serine acetyltransferase (CysE), preferably for a serine acetyltransferase having a sequence identity of at least 90, 95 or 98%, preferably 100%, to the sequence according to SEQ ID NO: 34,
s) an overexpressed polynucleotide (cysK), which codes for a cysteine synthase (CysK), preferably for a cysteine synthase having a sequence identity of at least 90, 95 or 98%, preferably 100%, to the sequence according to SEQ ID NO: 36,
t) an overexpressed polynucleotide (glyA), which codes for a serine hydroxymethyltransferase (GlyA), preferably for a serine hydroxymethyltransferase having a sequence identity of at least 90, 95 or 98%, preferably 100%, to the sequence according to SEQ ID NO: 44,
u) an overexpressed polynucleotide (hom), which codes for an optionally feedback-resistant homoserine dehydrogenase (Hom), preferably for a homoserine dehydrogenase having a sequence identity of at least 90, 95 or 98%, preferably 100%, to the sequence according to SEQ ID NO: 46,
v) an overexpressed polynucleotide (lysC), which codes for an optionally feedback-resistant aspartate kinase (LysC), preferably for an aspartate kinase having a sequence identity of at least 90, 95 or 98%, preferably 100%, to the sequence according to SEQ ID NO: 54,
w) an overexpressed polynucleotide (metB), which codes for a cystathionine gamma-synthase (MetB), preferably for a cystathionine gamma-synthase having a sequence identity of at least 90, 95 or 98%, preferably 100%, to the sequence according to SEQ ID NO: 56,
x) an overexpressed polynucleotide (metF), which codes for a 5,10-methylenetetrahydrofolate reductase (MetF), preferably for a 5,10-methylenetetrahydrofolate reductase having a sequence identity of at least 90, 95 or 98%, preferably 100%, to the sequence according to SEQ ID NO: 58,
y) an overexpressed polynucleotide (metX), which codes for a homoserine O-acetyltransferase (MetX), preferably for a homoserine O-acetyltransferase having a sequence identity of at least 90, 95 or 98%, preferably 100%, to the sequence according to SEQ ID NO: 60,
z) an overexpressed polynucleotide (metY), which codes for an O-acetylhomoserine lyase (MetY), preferably for an O-acetylhomoserine lyase having a sequence identity of at least 90, 95 or 98%, preferably 100%, to the sequence according to SEQ ID NO: 62,
aa) an overexpressed polynucleotide (pyc), which codes for a pyruvate carboxylase (Pyc), preferably for a pyruvate carboxylase having a sequence identity of at least 90, 95 or 98%, preferably 100%, to the sequence according to SEQ ID NO: 64,
bb) an overexpressed polynucleotide (serA), which codes for an optionally feedback-resistant D-3-phosphoglycerate dehydrogenase (SerA), preferably for a D-3-phosphoglycerate dehydrogenase having a sequence identity of at least 90, 95 or 98%, preferably 100%, to the sequence according to SEQ ID NO: 66,
cc) an overexpressed polynucleotide (serB), which codes for a phosphoserine phosphatase (SerB), preferably for a phosphoserine phosphatase having a sequence identity of at least 90, 95 or 98%, preferably 100%, to the sequence according to SEQ ID NO: 68,
dd) an overexpressed polynucleotide (serC), which codes for a phosphoserine aminotransferase (SerC), preferably for a phosphoserine aminotransferase having a sequence identity of at least 90, 95 or 98%, preferably 100%, to the sequence according to SEQ ID NO: 70,
ee) an overexpressed polynucleotide (ald), which codes for an alanine dehydrogenase (Ald), preferably for an alanine dehydrogenase having a sequence identity of at least 90, 95 or 98%, preferably 100%, to the sequence according to SEQ ID NO: 72,
ff) an overexpressed polynucleotide (cysD), which codes for the subunit of a sulphate adenylyltransferase (CysD), preferably for a subunit having a sequence identity of at least 90, 95 or 98%, preferably 100%, to the sequence according to SEQ ID NO: 74,
gg) an overexpressed polynucleotide (cysH), which codes for an adenosine phosphosulphate reductase (CysH), preferably for an adenosine phosphosulphate reductase having a sequence identity of at least 90, 95 or 98%, preferably 100%, to the sequence according to SEQ ID NO: 76,
hh) an overexpressed polynucleotide (cysI), which codes for a sulphite reductase (CysI), preferably for a sulphite reductase having a sequence identity of at least 90, 95 or 98%, preferably 100%, to the sequence according to SEQ ID NO: 78,
ii) an overexpressed polynucleotide (cysJ), which codes for (CysJ), preferably for one having a sequence identity of at least 90, 95 or 98%, preferably 100%, to the sequence according to SEQ ID NO: 80,
jj) an overexpressed polynucleotide (cysN), which codes for the subunit of a sulphate adenylyltransferase (CysN), preferably for a subunit having a sequence identity of at least 90, 95 or 98%, preferably 100%, to the sequence according to SEQ ID NO: 82,
kk) an overexpressed polynucleotide (cysY), which codes for a cystathionine beta-synthase (CysY), preferably for a cystathionine beta-synthase having a sequence identity of at least 90, 95 or 98%, preferably 100%, to the sequence according to SEQ ID NO: 84,
ll) an overexpressed polynucleotide (cysZ), which codes for a putative sulphate transporter (CysZ), preferably for a sulphate transporter having a sequence identity of at least 90, 95 or 98%, preferably 100%, to the sequence according to SEQ ID NO: 86,
mm) an overexpressed polynucleotide (metE), which codes for a 5-methyltetrahydropteroyltriglutamate-homocysteine methyltransferase (MetE), preferably for a protein having a sequence identity of at least 90, 95 or 98%, preferably 100%, to the sequence according to SEQ ID NO: 88,
nn) an overexpressed polynucleotide (ptH1), which codes for a peptidyl-tRNA hydrolase 1 (PtH1), preferably for a peptidyl-tRNA hydrolase 1 having a sequence identity of at least 90, 95 or 98%, preferably 100%, to the sequence according to SEQ ID NO: 90,
oo) an overexpressed polynucleotide (ptH2), which codes for a peptidyl-tRNA hydrolase 2 (PtH2), preferably for a peptidyl-tRNA hydrolase 2 having a sequence identity of at least 90, 95 or 98%, preferably 100%, to the sequence according to SEQ ID NO: 92.

11. Recombinant microorganism according to any of Claims 8 to 10, **characterized in that** said microorganism is a coryneform bacterium, particularly a Corynebacterium, preferably C. glutamicum or C. humireducens.

12. Method for overproducing an L-amino acid, **characterized in that** a glycine cleavage system according to any of Claims 1 to 4 and/or at least one polypeptide according to Claim 5 and/or at least one polynucleotide according to Claim 6 and/or a vector according to Claim 7 and/or a recombinant microorganism according to any of Claims 8 to 11 is used in said method.

13. Method according to the preceding claim, **characterized in that** the overproduced L-amino acid is selected from L-alanine, L-valine, L-amino acids of the glutamate family, particularly L-glutamate, L-glutamine, L-proline and L-arginine, and L-amino acids of the aspartate family, particularly L-aspartate, L-asparagine, L-methionine, L-lysine, L-isoleucine and L-threonine.

14. Method according to Claim 13, **characterized in that** the overproduced L-amino acid is L-methionine.

15. Method according to any of the preceding claims, **characterized in that** only low amounts of glycine occur as by-product.

## Revendications

1. Système de clivage de la glycine comprenant les enzymes GcvP, GcvT et GcvH, **caractérisé en ce qu'**il comprend au moins un des polypeptides suivants :
a) une enzyme GcvP ayant une identité de séquence d'au moins 80 % avec la séquence selon SEQ ID NO : 40,
b) une enzyme GcvT ayant une identité de séquence d'au moins 80 % avec la séquence selon SEQ ID NO : 42,
c) une enzyme GcvH ayant une identité de séquence d'au moins 80 % avec la séquence selon SEQ ID NO : 38.

2. Système de clivage de la glycine selon la revendication 1, **caractérisé en ce qu'**il comprend au moins deux, de préférence les trois polypeptides mentionnés précédemment.

3. Système de clivage de la glycine selon la revendication 2, **caractérisé en ce qu'**il comprend les polypeptides suivants :
a) une enzyme GcvP ayant une identité de séquence d'au moins 95 ou 98 % avec la séquence selon SEQ ID NO : 40,
b) une enzyme GcvT ayant une identité de séquence d'au moins 95 ou 98 % avec la séquence selon SEQ ID NO : 42,
c) une enzyme GcvH ayant une identité de séquence d'au moins 95 ou 98 % avec la séquence selon SEQ ID NO : 38.

4. Système de clivage de la glycine selon l'une quelconque des revendications précédentes, **caractérisé en ce qu'**il comprend au moins un polypeptide supplémentaire, choisi dans le groupe constitué par :
a) une enzyme LipA ayant une identité de séquence d'au moins 80 % avec la séquence selon SEQ ID NO : 48,
b) une enzyme LipB ayant une identité de séquence d'au moins 80 % avec la séquence selon SEQ ID NO : 50,
c) une enzyme Lpd ayant une identité de séquence d'au moins 80 % avec la séquence selon SEQ ID NO : 52,
d) une enzyme LplA ayant une identité de séquence d'au moins 80 % avec la séquence selon SEQ ID NO : 94,
e) une enzyme GcvL ayant une identité de séquence d'au moins 80 % avec la séquence selon SEQ ID NO : 96.

5. Polypeptides, qui sont des enzymes d'un système de clivage de la glycine, choisis dans le groupe constitué par :
a) une enzyme GcvP ayant une identité de séquence d'au moins 80 %, de préférence d'au moins 85 ou 90 %, notamment d'au moins 92, 94, 96 ou 98 %, avant tout de 100 %, avec la séquence selon SEQ ID NO : 40,
b) une enzyme GcvT ayant une identité de séquence d'au moins 80 %, de préférence d'au moins 85 ou 90 %, notamment d'au moins 92, 94, 96 ou 98 %, avant tout de 100 %, avec la séquence selon SEQ ID NO : 42,
c) une enzyme GcvH ayant une identité de séquence d'au moins 80 %, de préférence d'au moins 85 ou 90 %, notamment d'au moins 92, 94, 96 ou 98 %, avant tout de 100 %, avec la séquence selon SEQ ID NO : 38.

6. Polynucléotides, qui codent pour des enzymes d'un système de clivage de la glycine, choisis dans le groupe constitué par :
a) un polynucléotide (gcvP), qui code pour l'enzyme GcvP, et présente une identité de séquence d'au moins 70 ou 75 %, de préférence d'au moins 80 ou 85 %, notamment d'au moins 90 ou 95 %, avant tout d'au moins 98 ou 100 %, avec la séquence de la position 301 à 3 162 selon SEQ ID NO : 39, et/ou s'hybride dans des conditions stringentes avec un polynucléotide dont la séquence est complémentaire de la séquence de la position 301 à 3 162 selon SEQ ID NO : 39 ;
b) un polynucléotide (gcvT), qui code pour l'enzyme GcvT et présente une identité de séquence d'au moins 70 ou 75 %, de préférence d'au moins 80 ou 85 %, notamment d'au moins 90 ou 95 %, avant tout d'au moins 98 ou 100 %, avec la séquence de la position 301 à 1 404 selon SEQ ID NO : 41, et/ou s'hybride dans des conditions stringentes avec un polynucléotide dont la séquence est complémentaire de la séquence de la position 301 à 1 404 selon SEQ ID NO : 41 ;
c) un polynucléotide (gcvH), qui code pour l'enzyme GcvH et présente une identité de séquence d'au moins 70 ou 75 %, de préférence d'au moins 80 ou 85 %, notamment d'au moins 90 ou 95 %, avant tout d'au moins 98 ou 100 %, avec la séquence de la position 301 à 699 selon SEQ ID NO : 37, et/ou s'hybride dans des conditions stringentes avec un polynucléotide dont la séquence est complémentaire de la séquence de la position 301 à 699 selon SEQ ID NO : 37.

7. Vecteur, **caractérisé en ce qu'**il comprend au moins un polynucléotide, de préférence au moins deux ou trois polynucléotides, selon la revendication 6.

8. Microorganisme recombinant, **caractérisé en ce qu'**il comprend au moins un système de clivage de la glycine selon l'une quelconque des revendications 1 à 4 et/ou des polynucléotides codant pour un tel système de clivage de la glycine et/ou au moins un polypeptide selon la revendication 5 et/ou un polynucléotide codant pour un tel polypeptide ou des polynucléotides codant pour de tels polypeptides et/ou au moins un polynucléotide selon la revendication 6 et/ou un vecteur selon la revendication 7.

9. Microorganisme recombinant selon la revendication 8, **caractérisé en ce que** le système de clivage de la glycine et/ou les polynucléotides codant pour celui-ci se présentent sous forme surexprimée.

10. Microorganisme recombinant selon la revendication 8 ou 9, **caractérisé en ce qu'**il s'agit d'une souche de surproduction de L-méthionine et **en ce qu'**il présente au moins une, de préférence au moins deux ou trois, de manière particulièrement préférée quatre ou cinq, des caractéristiques suivantes :
a) un polynucléotide affaibli (mcbR), qui code pour un domaine de liaison à l'ADN de type HTH tetR (McbR), de préférence pour un domaine de liaison à l'ADN ayant une identité de séquence d'au moins 90, 95 ou 98 %, de préférence de 100 %, avec la séquence selon SEQ ID NO : 2,
b) un polynucléotide affaibli (gène thrB), qui code pour une homosérine-kinase (ThrB, CE 2.7.1.39), de préférence pour une homosérine-kinase ayant une identité de séquence d'au moins 90, 95 ou 98 %, de préférence de 100 %, avec la séquence selon SEQ ID NO : 4,
c) un polynucléotide affaibli (pgi), qui code pour une glucose-6-phosphate-isomérase (Pgi, CE 5.3.1.9), de préférence pour une glucose-6-phosphate-isomérase ayant une identité de séquence d'au moins 90, 95 ou 98 %, de préférence de 100 %, avec la séquence selon SEQ ID NO : 6,
d) un polynucléotide affaibli (pck), qui code pour une phosphoénolpyruvate-carboxykinase (Pck, CE 4.1.1.32), de préférence pour une phosphoénolpyruvate-carboxykinase ayant une identité de séquence d'au moins 90, 95 ou 98 %, de préférence de 100 %, avec la séquence selon SEQ ID NO : 8,
e) un polynucléotide affaibli (metQ), qui code pour une lipoprotéine qui se lie à la D-méthionine (MetQ), de préférence pour une lipoprotéine qui se lie à la D-méthionine ayant une identité de séquence d'au moins 90, 95 ou 98 %, de préférence de 100 %, avec la séquence selon SEQ ID NO : 10,
f) un polynucléotide affaibli (metP), qui code pour un transporteur de méthionine (MetP), de préférence pour un transporteur de méthionine ayant une identité de séquence d'au moins 90, 95 ou 98 %, de préférence de 100 %, avec la séquence selon SEQ ID NO : 12,
g) un polynucléotide affaibli (metN), qui code pour un transporteur de méthionine dépendant de l'ATP (MetN), de préférence pour un transporteur de méthionine dépendant de l'ATP ayant une identité de séquence d'au moins 90, 95 ou 98 %, de préférence de 100 %, avec la séquence selon SEQ ID NO : 14,
h) un polynucléotide affaibli (metK), qui code pour une S-adénosylméthionine-synthase (MetK), de préférence pour une S-adénosylméthionine-synthase ayant une identité de séquence d'au moins 90, 95 ou 98 %, de préférence de 100 %, avec la séquence selon SEQ ID NO : 16,
i) un polynucléotide affaibli (metI), qui code pour une perméase du système d'importation de méthionine (MetI), de préférence pour une perméase du système d'importation de méthionine ayant une identité de séquence d'au moins 90, 95 ou 98 %, de préférence de 100 %, avec la séquence selon SEQ ID NO : 18,
j) un polynucléotide affaibli (dapA), qui code pour une 4-hydroxy-tétrahydrodipicolinate-synthase (DapA), de préférence pour une 4-hydroxy-tétrahydrodipicolinate-synthase ayant une identité de séquence d'au moins 90, 95 ou 98 %, de préférence de 100 %, avec la séquence selon SEQ ID NO : 20,
k) un polynucléotide surexprimé (CBS), qui code pour une cystéine-synthase (CBS), de préférence pour une cystéine-synthase ayant une identité de séquence d'au moins 90, 95 ou 98 %, de préférence de 100 %, avec la séquence selon SEQ ID NO : 22,
l) un polynucléotide affaibli, qui code pour un homologue de cg3031, de préférence pour un homologue de cg3031 ayant une identité de séquence d'au moins 90, 95 ou 98 %, de préférence de 100 %, avec la séquence selon SEQ ID NO : 24,
m) un polynucléotide surexprimé (aecD), qui code pour une cystathionine-bêta-lyase (AecD), de préférence pour une cystathionine-bêta-lyase ayant une identité de séquence d'au moins 90, 95 ou 98 %, de préférence de 100 %, avec la séquence selon SEQ ID NO : 26,
n) un polynucléotide surexprimé (asd), qui code pour une aspartate-semi-aldéhyde-déshydrogénase (Asd), de préférence pour une aspartate-semi-aldéhyde-déshydrogénase ayant une identité de séquence d'au moins 90, 95 ou 98 %, de préférence de 100 %, avec la séquence selon SEQ ID NO : 28,
o) un polynucléotide surexprimé (metH), qui code pour une 5-méthyltétrahydrofolate-homocystéine-méthyltransférase (MetH, CE 2.1.1.13),
p) un polynucléotide surexprimé (brnE), qui code pour la sous-unité la plus petite d'un transporteur pour acides aminés à chaîne ramifiée (BrnE), de préférence pour une sous-unité ayant une identité de séquence d'au moins 90, 95 ou 98 %, de préférence de 100 %, avec la séquence selon SEQ ID NO : 30,
q) un polynucléotide surexprimé (brnF), qui code pour la sous-unité la plus grande d'un transporteur pour acides aminés à chaîne ramifiée (BrnF), de préférence pour une sous-unité ayant une identité de séquence d'au moins 90, 95 ou 98 %, de préférence de 100 %, avec la séquence selon SEQ ID NO : 32,
r) un polynucléotide surexprimé (cysE), qui code pour une sérine-acétyltransférase (CysE), de préférence pour une sérine-acétyltransférase ayant une identité de séquence d'au moins 90, 95 ou 98 %, de préférence de 100 %, avec la séquence selon SEQ ID NO : 34,
s) un polynucléotide surexprimé (cysK), qui code pour une cystéine-synthase (CysK), de préférence pour une cystéine-synthase ayant une identité de séquence d'au moins 90, 95 ou 98 %, de préférence de 100 %, avec la séquence selon SEQ ID NO : 36,
t) un polynucléotide surexprimé (glyA), qui code pour une sérine-hydroxyméthyltransférase (GlyA), de préférence pour une sérine-hydroxyméthyltransférase ayant une identité de séquence d'au moins 90, 95 ou 98 %, de préférence de 100 %, avec la séquence selon SEQ ID NO : 44,
u) un polynucléotide surexprimé (hom), qui code pour une homosérine-déshydrogénase éventuellement résistante à la rétroaction (Hom), de préférence pour une homosérine-déshydrogénase ayant une identité de séquence d'au moins 90, 95 ou 98 %, de préférence de 100 %, avec la séquence selon SEQ ID NO : 46,
v) un polynucléotide surexprimé (lysC), qui code pour une aspartate-kinase éventuellement résistante à la rétroaction (LysC), de préférence pour une aspartate-kinase ayant une identité de séquence d'au moins 90, 95 ou 98 %, de préférence de 100 %, avec la séquence selon SEQ ID NO : 54,
w) un polynucléotide surexprimé (metB), qui code pour une cystathionine-gamma-synthase (MetB), de préférence pour une cystathionine-gamma-synthase ayant une identité de séquence d'au moins 90, 95 ou 98 %, de préférence de 100 %, avec la séquence selon SEQ ID NO : 56,
x) un polynucléotide surexprimé (metF), qui code pour une 5,10-méthylène-tétrahydrofolate-réductase (MetF), de préférence pour une 5,10-méthylène-tétrahydrofolate-réductase ayant une identité de séquence d'au moins 90, 95 ou 98 %, de préférence de 100 %, avec la séquence selon SEQ ID NO : 58,
y) un polynucléotide surexprimé (metX), qui code pour une homosérine-O-acétyltransférase (MetX), de préférence pour une homosérine-O-acétyltransférase ayant une identité de séquence d'au moins 90, 95 ou 98 %, de préférence de 100 %, avec la séquence selon SEQ ID NO : 60,
z) un polynucléotide surexprimé (metY), qui code pour une O-acétylhomosérine-lyase (MetY), de préférence pour une O-acétylhomosérine-lyase ayant une identité de séquence d'au moins 90, 95 ou 98 %, de préférence de 100 %, avec la séquence selon SEQ ID NO : 62,
aa) un polynucléotide surexprimé (pyc), qui code pour une pyruvate-carboxylase (Pyc), de préférence pour une pyruvate-carboxylase ayant une identité de séquence d'au moins 90, 95 ou 98 %, de préférence de 100 %, avec la séquence selon SEQ ID NO : 64,
bb) un polynucléotide surexprimé (serA), qui code pour une D-3-phosphoglycérate-déshydrogénase éventuellement résistante à la rétroaction (SerA), de préférence pour une D-3-phosphoglycérate-déshydrogénase ayant une identité de séquence d'au moins 90, 95 ou 98 %, de préférence de 100 %, avec la séquence selon SEQ ID NO : 66,
cc) un polynucléotide surexprimé (serB), qui code pour une phosphosérine-phosphatase (SerB), de préférence pour une phosphosérine-phosphatase ayant une identité de séquence d'au moins 90, 95 ou 98 %, de préférence de 100 %, avec la séquence selon SEQ ID NO : 68,
dd) un polynucléotide surexprimé (serC), qui code pour une phosphosérine-aminotransférase (SerC), de préférence pour une phosphosérine-aminotransférase ayant une identité de séquence d'au moins 90, 95 ou 98 %, de préférence de 100 %, avec la séquence selon SEQ ID NO : 70,
ee) un polynucléotide surexprimé (ald), qui code pour une alanine-déshydrogénase (Ald), de préférence pour une alanine-déshydrogénase ayant une identité de séquence d'au moins 90, 95 ou 98 %, de préférence de 100 %, avec la séquence selon SEQ ID NO : 72,
ff) un polynucléotide surexprimé (cysD), qui code pour la sous-unité d'une sulfate-adénylyltransférase (CysD), de préférence pour une sous-unité ayant une identité de séquence d'au moins 90, 95 ou 98 %, de préférence de 100 %, avec la séquence selon SEQ ID NO : 74,
gg) un polynucléotide surexprimé (cysH), qui code pour une adénosine-phosphosulfate-réductase (CysH), de préférence pour une adénosine-phosphosulfate-réductase ayant une identité de séquence d'au moins 90, 95 ou 98 %, de préférence de 100 %, avec la séquence selon SEQ ID NO : 76,
hh) un polynucléotide surexprimé (cysI), qui code pour une sulfite-réductase (CysI), de préférence pour une sulfite-réductase ayant une identité de séquence d'au moins 90, 95 ou 98 %, de préférence de 100 %, avec la séquence selon SEQ ID NO : 78,
ii) un polynucléotide surexprimé (cysJ), qui code pour (CysJ), de préférence ayant une identité de séquence d'au moins 90, 95 ou 98 %, de préférence de 100 %, avec la séquence selon SEQ ID NO : 80,
jj) un polynucléotide surexprimé (cysN), qui code pour la sous-unité d'une sulfate-adénylyltransférase (CysN), de préférence pour une sous-unité ayant une identité de séquence d'au moins 90, 95 ou 98 %, de préférence de 100 %, avec la séquence selon SEQ ID NO : 82,
kk) un polynucléotide surexprimé (cysY), qui code pour une cystathionine-bêta-synthase (CysY), de préférence pour une cystathionine-bêta-synthase ayant une identité de séquence d'au moins 90, 95 ou 98 %, de préférence de 100 %, avec la séquence selon SEQ ID NO : 84,
ll) un polynucléotide surexprimé (cysZ), qui code pour un transporteur de sulfate putatif (CysZ), de préférence pour un transporteur de sulfate ayant une identité de séquence d'au moins 90, 95 ou 98 %, de préférence de 100 %, avec la séquence selon SEQ ID NO : 86,
mm) un polynucléotide surexprimé (metE), qui code pour une 5-méthyltétrahydroptéroyl-triglutamate-homocystéine-méthyltransférase (MetE), de préférence pour une protéine ayant une identité de séquence d'au moins 90, 95 ou 98 %, de préférence de 100 %, avec la séquence selon SEQ ID NO : 88,
nn) un polynucléotide surexprimé (ptH1), qui code pour une peptidyl-ARNt-hydrolase 1 (PtH1), de préférence pour une peptidyl-ARNt-hydrolase 1 ayant une identité de séquence d'au moins 90, 95 ou 98 %, de préférence de 100 %, avec la séquence selon SEQ ID NO : 90,
oo) un polynucléotide surexprimé (ptH2), qui code pour une peptidyl-ARNt-hydrolase 2 (PtH2), de préférence pour une peptidyl-ARNt-hydrolase 2 ayant une identité de séquence d'au moins 90, 95 ou 98 %, de préférence de 100 %, avec la séquence selon SEQ ID NO : 92.

11. Microorganisme recombinant selon l'une quelconque des revendications 8 à 10, **caractérisé en ce qu'**il s'agit d'une bactérie coryneforme, notamment d'une corynebactérie, de préférence de C. glutamicum ou C. humireducens.

12. Procédé de surproduction d'un acide L-aminé, **caractérisé en ce qu'**un système de clivage de la glycine selon l'une quelconque des revendications 1 à 4 et/ou au moins un polypeptide selon la revendication 5 et/ou au moins un polynucléotide selon la revendication 6 et/ou un vecteur selon la revendication 7 et/ou un microorganisme recombinant selon l'une quelconque des revendications 8 à 11 est utilisé dans ce procédé.

13. Procédé selon la revendication précédente, **caractérisé en ce que** l'acide L-aminé surproduit est choisi parmi la L-alanine, la L-valine, les acides L-aminés de la famille du glutamate, notamment le L-glutamate, la L-glutamine, la L-proline et la L-arginine, ainsi que les acides L-aminés de la famille de l'aspartate, notamment le L-aspartate, la L-asparagine, la L-méthionine, la L-lysine, la L-isoleucine et la L-thréonine.

14. Procédé selon la revendication 13, **caractérisé en ce que** l'acide L-aminé surproduit est la L-méthionine.

15. Procédé selon l'une quelconque des revendications précédentes, **caractérisé en ce que** seules de petites quantités de glycine se forment en tant que produit secondaire.
